# EUROPÄISCHE PATENTSCHRIFT

(11) **EP 1 427 837 B1**
(45) Veröffentlichungstag und Bekanntmachung des Hinweises auf die Patenterteilung: **26.10.2011**
(21) Anmeldenummer: 02704571.5
(22) Anmeldetag: 10.01.2002
(51) Int. Cl.: C12N 15/87, C07K 14/47, C07K 19/00, A61K 48/00, G01N 33/50, C12Q 1/68

(54) **MODULARE TRANSFEKTIONSSYSTEME AUF DER BASIS VON NUKLEOPROTEINFILAMENTEN**
MODULAR TRANSFECTION SYSTEMS BASED ON NUCLEOPROTEIN FILAMENTS
SYSTÈMES DE TRANSFECTION MODULAIRES À BASE DE FILAMENTS DE NUCLÉOPROTEINES

(30) Priorität: 10.01.2001 DE 10100996
(43) Veröffentlichungstag der Anmeldung: 16.06.2004
(73) Patentinhaber: Lonza Cologne GmbH, 50829 Köln (DE)
(72) Erfinder: SCHMIDT, Hanns-Martin, 50733 Köln (DE); ALTROGGE, Ludger, 50259 Pulheim (DE); LENZ, Dietmar, 50676 Köln (DE); RIEMEN, Gudula, 40764 Langenfeld (DE); BROSTERHUS, Helmut, 57399 Kirchundern (DE); LORBACH, Elke, 50677 Köln (DE); HELFRICH, Juliana, 51103 Köln (DE); HEIN, Katharina, 50678 Köln (DE); GREMSE, Marion, 50829 Köln (DE); MALES, Tatjana, 40237 Hilden (DE); CHRISTINE, Rainer, 50931 Köln (DE); SIEBENKOTTEN, Gregor, 50226 Frechen-Königsdorf (DE); ORTMANN, Bodo, 50678 Köln (DE); TURBANSKI, Tamara, 51674 Wiehl (DE); KLAES, Andrea, 50935 Köln (DE)
(74) Vertreter: Remus, Alvaro Johannes
(86) Internationale Anmeldenummer: PCT/DE2002/000060
(87) Internationale Veröffentlichungsnummer: WO 2002/055721

(56) Entgegenhaltungen:
- WO-A-95/34295
- DE-A- 19 925 052
- US-A- 4 950 599
- US-A- 5 468 629
- US-A- 5 763 240
- BERTOLOTTI R: "Recombinase-DNA nucleoprotein filaments as gene therapy vectors." BIOGENIC AMINES, Bd. 14, Nr. 1, 1998, Seiten 41-65, XP008017788 ISSN: 0168-8561
- KIDO M ET AL: "ESCHERICHIA COLI RECA PROTEIN MODIFIED WITH A NUCLEAR LOCATION SIGNAL BINDS TO CHROMOSOMES IN LIVING MAMMALIAN CELLS" EXPERIMENTAL CELL RESEARCH, SAN DIEGO, CA, US, Bd. 198, Nr. 1, 1992, Seiten 107-114, XP000616315 ISSN: 0014-4827

### SEQUENZPROTOKOLL

<110> amaxa GmbH
<120> Modulare Transfektionssysteme
<130> A02114PCT
<140> PCT-Anmeldung
   <141> 2002-01-10
<150> DE 101 00 996.8
   <151> 2001-01-10
<160> 1
<170> PatentIn Ver. 2.1
<210> 1
   <211> 14
   <212> PRT
   <213> Künstliche Sequenz
<220>
   <223> Beschreibung der künstlichen Sequenz:durchmischte Sequenz von nls-2 (Aminosäuren 2-15 von SEQ ID NO:9 aus WO 00/40742), d.h gleiche Aminosäuren, aber unterschiedliche Reihenfolge
<400> 1
   Glu Lys Pro Glu Lys Asp Lys Glu Pro Arg Thr Lys Val Asp
    1               5                  10

## Beschreibung

Die vorliegende Erfindung liegt auf dem Gebiet der nicht-viralen Transfektion von Nukleinsäuren. Unter dem Begriff Transfektion versteht man allgemein die Einbringung fremder Substanzen in Zellen. Die vorliegende Erfindung betrifft dabei ein Transfektionsagens enthaltend ein Nukleoproteinfilament, gebildet aus mindestens einer zu transfizierenden Nukleinsäure und mindestens einem zur Nukleoproteinfilamentbildung fähigen Protein. Die Erfindung betrifft ferner ein Verfahren zur *in vitro* Transfektion von Zellen mit Hilfe des Transfektionsagens. Des weiteren betrifft die vorliegende Erfindung die Verwendung des erfindungsgemäßen Transfektionsagens, eine entsprechende pharmazeutische Zubereitung, insbesondere zur Verwendung in der Gentherapie, sowie einen Kit zur Transfektion von Zellen mit Nukleinsäuren.

### Stand der Technik

Die bekannten nicht-viralen Transfektionsagenzien sind einer Reihe von Beschränkungen unterworfen. Bei der nicht-viralen Nukleinsäuretransfektion stellt meist sowohl die Größe der globulären und oft einseitig geladenen Nukleinsäurekomplexe wie auch die mangelnde Steuerbarkeit einzelner oder mehrerer Transfektionsschritte ein großes Problem dar. Letzteres ist unter anderem bedingt durch ihre mangelhafte Fähigkeit, die äußere Zellmembran oder innere Kompartimentmembranen zu durchdringen, mangelnden Schutz vor enzymatischem Abbau, geringe Bioverfügbarkeit sowie ungenügend kontrollierbare biologische Effekte nicht-biologischer Moleküle in der Zelle. Nukleinsäuren sind sterisch meist sehr ausladend, enzymatisch leicht abbaubar und assozieren durch ihren einseitigen Ladungsüberschuß leicht mit basischen Zellstrukturen. Die dadurch bedingte ungenügende Fähigkeit zur Kempassage führt dazu, dass derzeitige Nukleinsäuretransfektionstechnologien fast ausschließlich auf die Verwendung krebsartig entarteter Zellen, wie beispielsweise Zelllinien, beschränkt sind, da sich bei diesen Zellen die Kemmembran während der Zellteilung vorübergehend auflöst und ein Eintritt in den Kern möglich ist. Entartete Zellen sind aber mit dem ursprünglichen physiologischen Zustand primärer Zellen nicht vergleichbar, so dass aus entsprechenden Erkenntnissen mit solchen Zellen nicht ohne weiteres auf das Verhalten primärer transfizierter Zellen geschlossen werden kann.

Es sind bereits eine Reihe von Transfektionsagenzien bekannt, von denen die weitaus meisten durch elektrostatische Interaktion mit Nukleinsäuren globuläre Komplexe ausbilden, oft mit einem Durchmesser von deutlich mehr als 50 nm (Tang und Szoka, 1997). Diese Komplexe assoziieren meist mit hoher Überschußladung an der Zelloberfläche und werden endozytotisch aufgenommen. Sie verlassen die Endosomen entweder durch Abpuffern der endosomalen Ansäuerung bis sie platzen (z.B. mit Polyethylenimin, Starburst Dendrimeren (Kukowska-Latello et al. 1996) oder Zugabe von Chloroquin) oder durch die Wirkung membranaktiver Gruppen, Lipide oder lipophiler Peptide. Die Komplexe können jedoch erst bei der nächsten Zellteilung in den Zellkern gelangen, um dort nach der Nukleinsäurefreisetzung die gewünschte Wirkung zu entfalten.

Das Problem der teilungsabhängigen Kempassage kann durch Verwendung von NLS-Peptiden (NLS = nuclear localization signal = Kemlokalisationssignal) umgangen werden, vor allem, wenn der Problematik der Signalmaskierung und der unspezifischen DNA-Bindung Rechnung getragen wird (WO 00/40742, Amaxa).

Im US-Patent Nr. 5,468,629 wird die Verwendung des RecA-Proteins sowie die mögliche Verwendung weiterer RecA-funktionshomologer Proteine zur Transfektion von Zellen mit ssDNA (Einzelstrang-DNA) beschrieben. Das Protein RecA unterstützt dabei in offenbar katalytischer Weise den Vorgang der homologen Rekombination der transportierten ssDNA mit der Zell-DNA durch Beeinflussung der Strangpaarung und des anschließenden Strangaustausches. Die hier verwendeten Komplexe aus ssDNA von maximal 700 Nukleotiden und RecA-Protein sind als "RecA-coated" (mit RecA-Protein überzogene) Komplexe ausgeführt. Solche ssDNA-Protein-Komplexe werden durch DNA unter Zugabe von RecA in Anwesenheit von ATP-γ-S ausgebildet, wobei stabile helikale präsynaptische Filamente ausgebildet werden. Diese Komplexe werden beispielhaft zur Transfektion von Zellinien, d. h. für teilungsaktive Zellen, insbesondere entartete Zellen mit geringem Erfolg eingesetzt.

Bertolotti (Bertolotti, R. (1998): Recombinase-DNA nucleoprotein filaments as Gene Therapy vectors. Biogenic Amines, Vol. 14, No. 1, 41-65.) offenbart ein Verfahren zurm Gewebe-spezifischen DNA-Transfer mit Hilfe von Rekombinase-DNA-Nukleoproteinfilamenten. Es wird dabei vorgeschlagen, die Nukleoproteinfilamente entweder durch Glykosylierung der Rekombinase, Fusion mit kleinen Peptiden, Fusion mit Signalpeptiden (NLS) oder die Assoziierung mit endosomolytischen Faktoren oder Integrin-bindenden Peptiden (RGD) zu modifizieren.

### Zusammenfassung der Erfindung

Es ist Aufgabe der vorliegenden Erfindung, ein Verfahren und ein Transfektionsagens zur Transfektion von Nukleinsäuren jeder Art in Zellen jeder Art zur Verfügung zu stellen, die eine verbesserte Aufnahme in Zellen ermöglichen und gleichzeitig eine Steuerung des Transfektionsvorgangs erlauben. Das Transfektionsagens sollte dabei sowohl während des Transfektionsvorgangs ausreichend stabil sein, als auch eine ausreichende Freisetzung der zu transfizierenden Nukleinsäuren im Zielkompartiment gewährleisten.

Diese Aufgabe wird erfindungsgemäß durch ein Transfektionsagens der eingangs genannten Art gelöst, bei dem das zur Nukleoproteinfilamentbildung fähige Protein mit mindestens zwei funktionellen Komponenten unterschiedlicher Funktion modifiziert ist, wobei eine funktionelle Komponente ein Kernlokalisationssignal ist und mindestens eine weitere funktionelle Komponente ausgewählt ist aus der Gruppe bestehend aus einem VP22-Peptid und einem Integrin-bindenden RGD-Motiv.

Die Aufgabe wird erfindungsgemäß ferner durch ein Transfektionsagens enthaltend ein Nukleoproteinfilament, gebildet aus mindestens einer zu transfizierenden Nukleinsäure und zur Nukleoproteinfilamentbildung fähigen Proteinen gelöst, bei dem mindestens zwei gleiche oder unterschiedliche, zur Nukleoproteinfilamentbildung fähige Proteine mit einer Komponente unterschiedlicher Funktion modifiziert sind, wobei eine funktionelle Komponente ein Kernlokalisationssignal ist und mindestens eine weitere funktionelle Komponente ausgewählt ist aus der Gruppe bestehend aus einem VP22-Peptid und einem Integrin-bindenden RGD-Motiv.

Die Erfindung umfasst auch ein Verfahren zur *in vitro* Transfektion von Zellen mit Hilfe zumindest eines der oben definierten Transfektionsagenzien, bei dem das Transfektionsagens auf die zu transfizierenden Zellen gegeben wird.

Durch die Modifikation einzelner oder mehrerer Nukleoproteinfilament (NPF) - bildender Proteine zumindest mit mindestens einem Kernlokalisationssignal sowie mindestens einem VP22-Peptid und/oder mindestens einem Integrin-bindenden RGD-Motiv können einzelne Schritte des komplexen Transfektionsvorgangs in besonders vorteilhafter Weise individuell für die zu transfizierende Nukleinsäure und die Zielzelle gesteuert werden. Durch das erfindungsgemäße Verfahren bzw. Transfektionsagens läßt sich ferner die Transfektionseffizienz gegenüber bekannten Verfahren deutlich steigern.

Erfindungsgemäß werden NPF-bildende Proteine in vorteilhafter Weise über die reine Schutzfunktion für die DNA hinaus, vor allem als modulares Trägersystem für funktionelle Gruppen eingesetzt. Es zeigt in zweierlei Hinsicht eine extrem geringe Raumausdehnung: es ist filamentös - nicht globulär - und es enthält nur ein Nukleinsäuremolekül pro Filament. Aufgrund des Aufbaus, der zu einem stöchiometrischen Verhältnis von wenigen Nukleotiden pro Trägerprotein führt, ist eine extrem hohe Dichte an funktionellen Signalen für die Transfektion möglich, deutlich mehr als bei globulären Komplexen. Die Signaldichte sowie Kombinationen verschiedener Signale sind durch Mischung verschiedener funktionalisierter Trägerproteine und Proteine ohne funktionelle Gruppen individuell einstellbar, so dass das erfindungsgemäße Transfektionsagens als modulares System auf unterschiedlichste Transfektionsbedingungen ausgerichtet werden kann. Die geringe Raumausdehnung und die hohe Signaldichte ermöglichen es zudem, zusätzlich zelleigene, auf kleine Moleküle ausgelegte Transportsysteme für die Transfektion zu nutzen. Aufgrund seines filamentösen Charakters, seines einfachen Aufbaus, seines geringen Durchmessers und seiner einstellbaren, extrem hohen Signaldichte ermöglicht das erfindungsgemäße Verfahren bzw. ist das erfindungsgemäße Transfektionsagens in der Lage, auch große Nukleinsäuremoleküle, wie z.B. Expressionsvektoren, unter Ausnutzung zelleigener Mechanismen gezielt zu transportieren.

Die zusätzlichen funktionellen Komponenten können dabei beispielsweise direkt an die NPF-Proteine gebunden werden oder über einen Spacer, einen nicht funktionellen Abstandshalter. Solche Spacer bewirken einen größeren Abstand von NPF-Protein und funktioneller Komponente, der eine gegenseitige sterische Hinderung vermeidet und eine bessere räumliche Verfügbarkeit des NPF-Proteins und der funktionellen Komponente gewährleistet. Die Struktur der erfindungsgemäßen Agenzien verhindert dabei weitgehend eine Maskierung der funktionellen Komponenten.

NPF-bildende Proteine bilden mit Nukleinsäuren meist durch kooperative Bindung Nukleoproteinfilamente, in denen Proteine und Nukleinsäuren einen Komplex eingehen, dessen Größe bzw. Durchmesser im Vergleich zu den bekannten globulären Transfektionsagenzien erheblich verringert ist. Ein solches NPF kann z. B. von den Proteinen der RecA-Familie, die DNA-abhängige ATPasen sind, in Gegenwart von Nukleosidtriphosphaten wie ATP (Adenosintriphosphat) gebildet werden, bei vollständiger Beladung mit einer Dichte von z. B. einem Protein pro drei Basenpaaren bei doppelsträngiger DNA (Bianco et al., 1998). Diese hohe Proteindichte bietet einen ausgezeichneten Schutz gegenüber der enzymatischen Hydrolyse der Nukleinsäure, da enzymatische Angriffspunkte stark reduziert werden.

Die NPF vermitteln zum einen eine ausreichende Stabilität des Komplexes und ermöglichen zum anderen eine genügende Freisetzung der transportierten Nukleinsäuren, z. B. im Kern. Durch Verwendung nicht- oder schwer hydrolysierbarer Analoga von Nukleosidtriphosphaten, beispielsweise von ATP und/oder GTP, wie z. B. ATP-γ-S oder GTP-γ-S, besteht zudem die Möglichkeit, NPFs, die mit Hilfe ATPbindender Proteine, wie die der RecA-Familie gebildet werden, zusätzlich zu stabilisieren.

Die zur NPF-Bildung fähigen Proteine können auch derivatisiert werden. Beispielsweise können Fusionsproteine hergestellt werden. Des weiteren können die NPF-bildenden Proteine verkürzt oder verlängert werden, einzelne Abschnitte oder Aminosäuren deletiert, eingefügt oder kovalent chemisch modifiziert werden, solange sie ihre erfindungswesentliche Funktion, die strukturelle Ausbildung von NPF mit Nukleinsäuren, wahrnehmen können.

Ein besonderer Vorteil ist erfindungsgemäß in der räumlichen Struktur der NPFs zu sehen, die es möglich macht, die natürlichen Kerntransportmechanismen auszunutzen. Der durch die Kernporengröße bedingte maximale Durchmesser des Transfektionsagens wird auch bei sehr langen Nukleinsäuren nicht überschritten, d.h. NPFs können unabhängig von der Länge der zu transfizierenden Nukleinsäure als Transfektionsagens eingesetzt werden. Es ist gezeigt worden, dass die Größenbegrenzung für den Transport durch die Kempore bei ca. 25 nm (Feldherr und Akin, 1997) bzw. 50 nm (SV40-Virus) liegt (Yamada und Kasamatsu, 1993). Nukleinsäuren, denen der Kernmembrandurchtritt mit herkömmlichen Transfektionsagenzien mit globulären Strukturen und/oder unspezifischer Bindung von mehreren Nukleinsäuremolekülen pro Transfektionskomponente versperrt ist, können eine solche Grenze mit Transfektionsagenzien im Sinne der vorliegenden Erfindung leicht unterschreiten. Die Struktur der erfindungsgemäßen Transfektionsagenzien ermöglicht erstmals sogar Durchmesser von ≤ 11 nm, je nach verwendetem NPF-bildendem Protein. Eine durch Verwendung von NPF-Proteinen fadenförmig aufgebaute Struktur ist daher auch geeignet für den Transport längerer Nukleinsäuren von mehreren Kilobasen Länge. Das erfindungsgemäße Verfahren und die erfindungsgemäßen Transfektionsagenzien eignen sich folglich besonders gut zur Transfektion von Zellen mit größeren Nukleotidsequenzen. Bevorzugt sind die erfindungsgemäßen Transfektionsagenzien, die mindestens eine zu transfizierende Nukleinsäure von mindestens 700 Nukleotiden enthalten.

Die vorliegende Erfindung kann vorteilhafterweise als solche zur Transfektion von Nukleinsäuren eingesetzt werden oder auch in Kombination mit anderen Transfektionsmethoden und Mitteln. Der hohe Beladungsgrad mit NPF-bildenden Proteinen erhöht die Hydrolysebeständigkeit und der geringe Durchmesser der NPF ermöglicht die Ausnutzung zelleigener Transportsysteme, die nur ausreichend kleinen Molekülen zugänglich sind, wie z. B. des Kerntransportsystems. Zudem ist die ausreichende Freisetzung der zu transfizierenden Nukleinsäuren in den Zellkompartimenten, vorzugsweise im Kern, gewährleistet.

Der erfindungsgemäße Begriff "Transfektionsagens" ist als Transportvehikel für Nukleinsäuren bzw. deren Derivate zu verstehen, das die zu transfizierende Nukleinsäure bereits enthält. Ein Transfektionsagens im Sinne dieser Erfindung führt mindestens zwei einzelne Schritte des komplexen Transfektionsvorgangs aus.

Im Rahmen der vorliegenden Erfindung bedeutet der Begriff "Nukleoproteinfilament" (NPF) eine molekulare Struktur, bestehend aus Nukleinsäure(n) oder Nukleinsäurederivaten und Proteinen, die durch nicht-kovalente, meist kooperative Bindung einen filamentösen bzw. fadenförmigen Komplex bilden, der bevorzugt nur ein einziges Nukleinsäuremolekül oder Derivat davon enthält. Besonders bevorzugt sind helikale Nukleoproteinfilamente, wie sie z. B. von RecA mit einzel- oder doppelsträngiger DNA gebildet werden (Di Capua et al., 1982).

Die zu "transfizierende Nukleinsäure" kann sowohl eine doppel- oder einzelsträngige DNA, eine doppel- oder einzelsträngige RNA sowie eine doppelsträngige DNA mit einzelsträngigen Enden sein, ein DNA/RNA-Hybrid, eine Antisense-DNA, Antisense-RNA oder chemisch modifizierte Nukleinsäurederivate, bei denen beispielsweise die Hydrolysebeständigkeit erhöht wurde (peptide nucleic acid, PNA), oder in die reaktive Molekülgruppen zur kovalenten Bindung und/oder Veränderung von Zielnukleinsäuren eingefügt wurden. Unter Derivaten der transfizierbaren Nukleinsäuren wird auch die Modifizierung der verwendeten Nukleinsäure durch ein sequenzspezifisch oder kovalent gebundenes Protein verstanden. Die bevorzugte Nukleinsäure der vorliegenden Erfindung ist DNA, insbesondere doppelsträngige DNA. NPF-bildende Proteine wurden bisher zumeist im Zusammenhang mit einzelsträngiger DNA, beispielsweise zur Rekombination, verwendet. Überraschenderweise hat sich aber im Rahmen der Erfindung herausgestellt, dass viele NPF-bildende Proteine unter geeigneten Bedingungen auch mit doppelsträngiger DNA stabile Komplexe bilden und zur erfindungsgemäßen Transfektion von doppelsträngiger DNA verwendet werden können. In der Möglichkeit auch doppelsträngige DNA effizient zu transfizieren, liegt daher ein weiterer Vorteil der Erfindung.

Die erste Hürde einer Transfektion besteht in der Assoziation des Transfektionskomplexes an die Zelloberfläche. Bei dem erfindungsgemäßen Transfektionsagens kann daher mindestens ein zur NPF-Bildung fähiges Protein verwendet werden, das mit mindestens einer funktionellen Gruppe modifiziert ist, die die Assoziation des Komplexes bzw. Agens an die Zelloberfläche bewirkt, nämlich mit dem Integrin/Integrin-bindenden Peptid RGD (Harbottle 1998).

Erfindungsgemäß kann die funktionelle Komponente auch den nicht-endosomalen Durchtritt des Komplexes bzw. Agens durch die Zellmembran bewirken. Der nichtendosomale Membrandurchtritt hat den Vorteil, dass das Agens gleich im Zytosol verfügbar ist und nicht der hydrolytisch aktiven Umgebung der Lysosomen ausgesetzt ist. Ein solcher Membrandurchtritt wird erfindungsgemäß durch das membranaktive virale Peptid VP22 erreicht.

Eine besondere Hürde stellt für alle Transfektionsagenzien der Import in den Kern dar, welcher gewöhnlich abhängig von der Zellteilung, ggf. nach Zellstimulation erfolgt. Erfindungsgemäß ist daher eine funktionelle Komponente vorgesehen, die den Transport des Komplexes bzw. Agens in den Zellkern bewirkt. Wesentlich für den Kerntransport sind zum einen der limitierende Kemporendurchmesser, zum anderen die für den Transport verwendeten Signalmoleküle. Als Signale für den Kemtransport im Rahmen der Erfindung dienen NLS (nuclear localization signals, Kernlokalisationssignale). Bevorzugt finden als Kernlokalisationssignale solche Signalsequenzen Anwendung, die entweder als solche und/oder zusammen mit ihren flankierenden Regionen keinen oder nur einen geringen positiven Ladungsüberschuß aufweisen, da ein Ladungsüberschuß zu unspezifischer Nukleinsäurebindung und somit zur Maskierung des Signals führen kann. Gut geeignet sind erweiterte Sequenzen sogenannter klassischer NLS, wenn die Summenladung des Peptids durch flankierende negativ geladene Aminosäuren zumindest annähernd ausgeglichen werden kann. Diese Aminosäuren können im Peptid/Protein an diesen Positionen natürlicherweise vorkommen oder aber aufgrund struktureller Überlegungen dort eingeführt werden. Auch sogenannte nichtklassische-NLS, wie z. B. ein NLS aus dem Influenza Virus "nucleoprotein" (Wang et al., 1997, Neumann et al., 1997) oder die Sequenz M9 aus dem heterogenen nuklearen RNP (hnRNP) A1 Protein, die keinen großen Überschuß an positiven Ladungen besitzen oder nicht über den klassischen Transportweg in den Kern gelangen, können Verwendung finden. Eine nicht vollständige, aber gute Übersicht über NLS, die zur Ausführung der vorliegenden Erfindung Anwendung finden können, gibt T. Boulikas (1993, 1996, 1997). Annähernd ladungsneutrale Sequenzen sind z. B. die NLS aus dem großen T-Antigen des Simian Virus 40 mit flankierenden natürlich vorkommenden oder künstlich eingeführten negativ geladenen Aminosäuren (siehe auch WO 00/40742 Amaxa).

Ein besonderer Vorteil der Erfindung liegt daher in der deutlichen Verbesserung der Transfektion von schwach oder nicht-teilungsaktiven eukaryontischen Zellen, insbesondere primären eukaryontischen Zellen mit Nukleinsäuren. Gerade die zuletzt genannten Zellen, die ihre biologische und medizinische Aussagekraft noch nicht eingebüßt haben, da sie beispielsweise unmittelbar aus einem Körper mittels Blutprobe oder Gewebebiopsie entnommen wurden, sind von entscheidender Bedeutung für den Fachmann. Bei der anstehenden Analyse der fast vollständigen Entschlüsselung des menschlichen Genoms liefert letztendlich nur die gezielte Expression des zu untersuchenden Gens in Zellsystemen mit ausreichender physiologischer Aussagekraft klare Hinweise auf eine mögliche technische Nutzbarkeit. Zudem ist die Transfektion primärer Zellen eine essentielle Voraussetzung für die nicht-virale Gentherapie *ex vivo* und *in vivo.*

Die erfindungsgemäßen Transfektionsagenzien können, wie oben beschrieben, durch eine Vielzahl von zur NPF-Bildung fähigen Proteinen oder Derivaten davon realisiert werden, die wiederum in ursprünglicher oder funktionell modifizierter Form vorliegen können. Es können entweder nur ein oder mehrere verschiedene zur NPF-Bildung fähige Proteine verwendet und diese, je nach transfektionsspezifischer Anforderung, mit mindestens zwei oder mehreren funktionellen Komponenten unterschiedlicher Funktion modifiziert werden. Je nach schwerpunktmäßiger Zielsetzung können vom Anwender auf diese Art und Weise verschiedene Module zusammen gestellt werden. So entsteht ein Baukastensystem, dem unmodifizierte und modifizierte NPF-Proteine gleicher oder verschiedener Modifikation entnommen werden können, um eine Transfektionsstrategie optimal an die zu transfizierende Nukleinsäure und die Zielzellen anzupassen.

In besonders vorteilhafter Ausgestaltung der Erfindung ist vorgesehen, dass das Protein mit einer Vielzahl funktioneller Komponenten modifiziert wird bzw. ist. Hierdurch kann die Signaldichte insgesamt noch weiter erhöht werden, so dass verbesserte Transfektionseffizienzen und eine bessere Steuerung der Transfektion ermöglicht werden.

Ein großer Teil der NPF-bildenden Proteine bildet die NPF-Struktur in Anwesenheit von Nukleosidtriphosphaten wie ATP als Kofaktor aus. Die Zugabe von Nukleosidtriphosphaten bewirkt dabei eine Stabilisierung der NPF. Beim erfindungsgemäßen Verfahren können daher in vorteilhafter Weise durch Nukleosidtriphosphate und/oder nicht-hydrolysierbare Analoga davon, insbesondere durch ATP (Adenosintriphosphat) und/oder GTP (Guanosintriphosphat) und/oder deren nicht-hydrolysierbare Analoga ausgebildet bzw. stabilisiert werden. Dies geschieht gegebenenfalls durch kovalente Bindung nach photochemischer Reaktion. Nicht-hydrolysierbare Nukleosidtriphosphat-Analoga sind beispielsweise ATP-γ-S (Adenosin 5'-O-3-thiotriphosphat) und GTPγS (Guanosin 5'-O-3-thiotriphosphat) (Ellouze, 1999). Mögliche ATP-Analoga, die NPF-ATPasen nach photochemischer Reaktion modifizieren, sind 8N3ATP (8-Azidoadenosin 5'-triphosphat) bzw. 5'FSBA (5'-p-Fluorosulfonylbenzoyladenosin) (Knight, 1985).

In vorteilhafter Ausgestaltung des erfindungsgemäßen Verfahrens wird dieses in Kombination mit anderen biologischen und/oder chemischen und/oder physikalischen Transfermethoden für biologisch aktive Moleküle wie beispielsweise Liposomenvermittelter Transfer, Mikroinjektion, Elektroporation, Immunoporation, ballistische Methode, Transfer mit Hilfe kationischer Lipide, Kalziumphosphat, DEAE-Dextran, Polyethylenimin oder pH-sensitivem Hydrogel angewendet. Solche Transfermethoden, insbesondere die Elektroporation, können vorteilhaft einzelne Schritte des Transfektionsprozesses wie z. B. den Eintritt in das Zytoplasma der Zelle unterstützen.

Bevorzugte erfindungsgemäße Transfektionsagenzien enthalten als NPF-bildendes Protein ein Protein, ausgewählt aus der Gruppe der Proteine RecA, RadA, UvsX, ScRad51, RAD51, hDmc1, SASP, ICP8, vorzugsweise UvsX, besonders bevorzugt hRAD51oder eine Mischung aus mindestens 2 der aufgezählten Proteine oder ein oder mehrere Derivate dieser Proteine. NPF-bildende Proteine zur Ausführung der vorliegenden Erfindung sind beispielsweise RecA aus *Escherichia coli* sowie seine Funktionshomologen aus Viren, Prokaryonten und Eukaryonten, wie z.B. UvsX aus dem Bakteriophagen T4 (Mosig, 1987) RadA aus Archaebakterien (Seitz et al., 1998), ScRad51 aus *Saccharomyces cerevisiae,* RAD51 aus Säugern und insbesondere hRad51 des Menschen. Homologe Proteine zu RecA sind in mindestens 60 verschiedenen Bakterienarten (Roca und Cox, 1990, Karlin und Brocchieri, 1996, Karlin et al., 1995), in Archaea (Sandler et al. 1996), in allen untersuchten Eukaryonten (Ogawa et al. 1993) sowie in Mitochondrien (Thyagarajan et al., 1996) und Plastiden (Cerutti et al., 1992) nachgewiesen worden. RecA und seine Homologen bilden mit einzel- und/oder doppelsträngiger DNA helikale NPF mit einem Durchmesser von ca. 11 nm. Die Bindung von RecA erfolgt kooperativ und führt zu einer teilweisen Entwindung der DNA-Helix. RecA, UvsX, ScRad51 und hRad51 bilden NPF mit einer Bindungsstöchiometrie von drei Basenpaaren pro Monomer (doppelsträngige DNA) bzw. 3 - 6 Nukleotiden pro Monomer (einzelsträngige DNA) (Bianco et al. 1998, Baumann und West 1998). Bevorzugt ist auch die Meiose-spezifische Rekombinase hDMC1, die mit doppelsträngiger DNA Filamente bildet, die aus einer linearen Reihe aus gestapelten Proteinringen besteht (Masson et al., 1999). Des weiteren ist auch die Gruppe der SASP-Proteine (small acid-soluble spore proteins) aus Sporen von *Bacillus* und *Clostridium* Arten bevorzugt. Auch die SASP binden an doppelsträngige DNA und bilden dabei helikale NPF mit einem Durchmesser von ca. 6,6 nm aus (Griffith et al. 1994). Auch virale Proteine, die mit einzel- und/oder doppelsträngiger DNA Filamente bilden können, wie z. B. das Protein ICP8 aus *Herpes simplex,* sind bevorzugte NPF-bildende Proteine (Lee & Knipe, 1985).

Für die NPF-bildenden Proteine UvsX, Rad51 und RecA wurde bei einer vollständigen Beladung von doppelsträngiger DNA eine Bindungsstöchiometrie von einem Monomer dieser Proteine pro 3 Basenpaaren gezeigt (Bianco et al 1998). Für UvsX z. B. kann solch ein vollständiger Beladungsgrad abhängig vom verwendeten Bindungspuffer, der Konformation der verwendeten Nukleinsäure und der Temperatur bei einem 3- bis 5-fachen Überschuss von Protein erreicht werden (Yu und Egelman 1993), beim α/β Typ SASP aus Bacillus subtilis bei einem Protein:DNA Verhältnis von ca. 5:1 (Griffith et al 1994). Ein solcher möglichst vollständiger Beladungsgrad der Nukleinsäuren mit Protein, der spezifisch für jedes der bevorzugten NPF-bildenden Proteine definiert ist, ist im Rahmen dieser Erfindung besonders bevorzugt. Aber auch eine Beladung der Nukleinsäuren unterhalb des höchstmöglichen Beladungsgrades für ein spezielles NPF-bildendes Protein ist im Rahmen der Erfindung möglich. Eine geringere Beladung der Nukleinsäuren mit NPF-bildenden Proteinen ist beispielsweise dann bevorzugt, wenn zusätzlich spezifisch an DNA bindende Proteine für definierte Schritte der Transfektion eingesetzt werden oder wenn für spezielle Anwendungen Pufferbedingungen gewählt werden müssen, die für eine vollständige Beladung der Nukleinsäure mit NPF-bildenden Proteinen nicht optimal sind.

Unter NPF-bildenden Proteinen sind sowohl natürliche NPF-bildende Proteine wie auch deren Derivate zu verstehen. Unter Derivaten von zur NPF-Bildung fähigen Proteinen versteht der Fachmann zum einen die Modifikation durch die Deletion oder Einführung zusätzlicher Aminosäureabfolgen bzw. Proteindomänen bei rekombinanten Proteinen und/oder die Einführung funktioneller Gruppen durch chemische Modifikation bereits vorhandener Molekülgruppen und/oder die chemische Kopplung von Proteinen, Peptiden, Kohlenhydraten, Lipiden oder anderen Molekülen.

Das erfindungsgemäße Transfektionsagens kann in vorteilhafter Weise auch zur Herstellung von Medikamenten zur gentherapeutischen Behandlung von Mensch und Tier verwendet werden. Die therapeutisch nutzbaren Nukleinsäuren können in Form der erfindungsgemäßen Transfektionagenzien auch primären Zellen und komplexen Transfektionsverläufen zugänglich gemacht werden.

Ein weiterer Aspekt der vorliegenden Erfindung betrifft daher pharmazeutische Zubereitungen, die ein erfindungsgemäßes Transfektionsagens, gegebenenfalls neben üblichen Hilfs- und Trägerstoffen enthalten.

Ein weiterer Aspekt der vorliegenden Erfindung betrifft Kits, die zur Transfektion von Zellen mit Nukleinsäuren geeignet sind und die modifizierte zur NPF-Bildung fähige Proteine wie oben definiert sowie mindestens eine der folgenden Komponenten umfassen:
a) Nukleosidtriphosphat und/oder Nucleosidtriphosphat-Analoga
b) mindestens eine zu transfizierende Nukleinsäure
c) Hilfs- und Zusatzstoffe

Solche Kits können individuell auf verschiedene Bedürfnisse von Fachleuten ausgerichtet sein, beispielsweise für bestimmte Nukleinsäuren, Zielzellen oder Stablitätsanforderungen durch die Auswahl spezifischer NPFs, spezifischer NPF-Modifikationen und einzelner Hilfs- und Zusatzstoffe optimiert sein. Dabei kann das Protein bereits mit der (den) funktionellen Komponente(n) beladen sein oder Proteine und funktionelle Komponenten können im Kit separat enthalten sein.

Die erfindungsgemäßen Transfektionsagenzien können beispielsweise zum Zellscreening verwendet werden, sprich zur Identifizierung von Aktivatoren oder Inhibitoren des oder der Expressionprodukt(e) des Transfektionsagens in mitotisch nicht oder schwach teilungsaktiven Zellen, primären Zellen und anderen Zellen mit begrenzter Lebensspanne. Solche Screenings stellen ein grundsätzliches Verfahren zur Identifizierung von Aktivatoren oder Inhibitoren validierter und nicht-validierter Targetproteine zur Wirkstofffindung in der pharmazeutischen Industrie dar. Diese Screeningverfahren sind meist so ausgestaltet, dass stabil mit Fremdnukleinsäuren transfizierte Zellen potentiellen Inhibitoren und/oder Aktivatoren ausgesetzt werden und deren Einfluß auf die Physiologie der Zellen, gegebenenfalls im Vergleich zu Vergleichszellen, bestimmt wird. Das erfindungsgemäße Transfektionsagens ist geeignet, die Fremdnukleinsäure kurz vor der Zugabe der Aktivatoren/Inhibitoren auch in mitotisch nicht oder schwach aktive Zellen, primäre Zellen und andere Zellen mit begrenzter Lebenspanne einzuführen und so diese Zellen als Testzellen zugänglich zu machen.

Die erfindungsgemäßen Transfektionsagenzien können ferner zur Identifizierung von physiologisch aktiven Nukleinsäuren verwendet werden. Dies ist insbesondere für die schnelle und physiologisch relevante Auswertung der Genomdatenmengen von Bedeutung, die der wissenschaftlich-pharmazeutischen Fachgemeinschaft zur Verfügung stehen. Dadurch, dass mit dem erfindungsgemäßen Transfektionsagens die Transfektion unabhängig von der Zellteilung und/oder der Endozytose wird, wird die Zeit zwischen Transfektion und Analyse erheblich verkürzt. Das ermöglicht einen deutlich höheren Probendurchsatz. Auch schwer transfizierbare, sogar nichtteilungsaktive primäre Zellen sind mit Hilfe der erfindungsgemäßen Transfektionsagenzien zugänglich. Die so erreichte Transfektion und die physiologische Auswertung von Veränderungen im

Vergleich zu Kontrollzellen ermöglicht die Identifizierung physiologisch aktiver Nukleinsäuren.

### Abkürzungen:

Außer den im Duden gebräuchlichen, wurden folgende Abkürzungen verwendet:
- AMP-PCP: Adenylyl-(β,g-methylen)-diphosphonat
- AMP-PNP: S'-Adenylylimido-diphosphat
- DEAE: Diethylaminoethan
- DNA: Desoxyribonukleinsäure
- dYT: double Yeast-Trypton
- FACScan: Fluorescence activated cell scanning
- FCS: foetales Kälberserum
- FL: Fluoreszenz
- FSC: Forwardscatter
- GMP-PNP: S'-Guanylylimido-diphosphat
- GTP: Guanosintriphosphat
- H6: Histidin-Hexamer
- Ig: Immunglobulin
- kb: Kilobasen
- ml: Milliliter
- mM: Millimol
- msec: Millisekunde
- NCBI: National Center for Biology Information
- ng: Nanogramm
- nm: nanomolar
- PBS: Phosphat-gepufferte Salzlösung
- PCR: Polymerase-Kettenreaktion
- Pi: Natriumdihydrogenphosphat/Dinatriumhydrogenphosphat
- RNA: Ribonukleinsäure
- RPMI: Roswell Park Memorial Institute
- SDS: Natriumdodecylsulfat
- SV40: Simian Virus 40
- TAE: Tris Acetat Ethylendiamintetraacetat
- U/mg: Einheiten/Milligramm
- upm: Umdrehungen pro Minute
- ZI-Puffer: Zellinjektions-Puffer
- µg: Mikrogramm
- µl: Mikroliter

### Kurze Beschreibung der Abbildungen

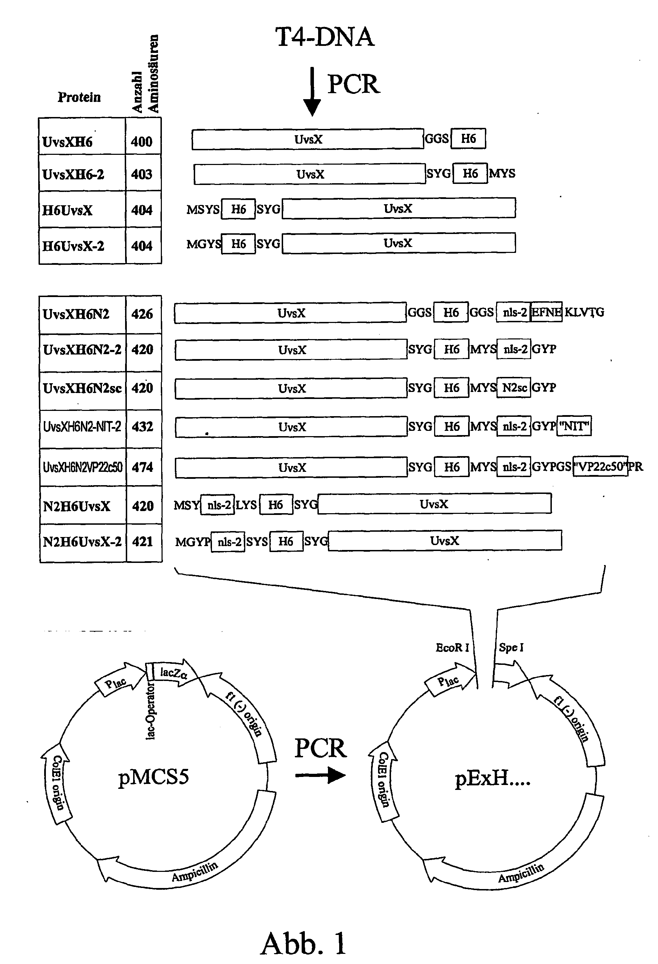

### Abbildung 1: Schematische Darstellung der Expressionsplasmide

Abb. 1 zeigt die schematische Darstellung des Aufbaus und der Herstellung der in den Beispielen beschriebenen NPF-bildenden Proteine.

### Abbildung 2: Bindung von UvsX an doppelsträngige DNA

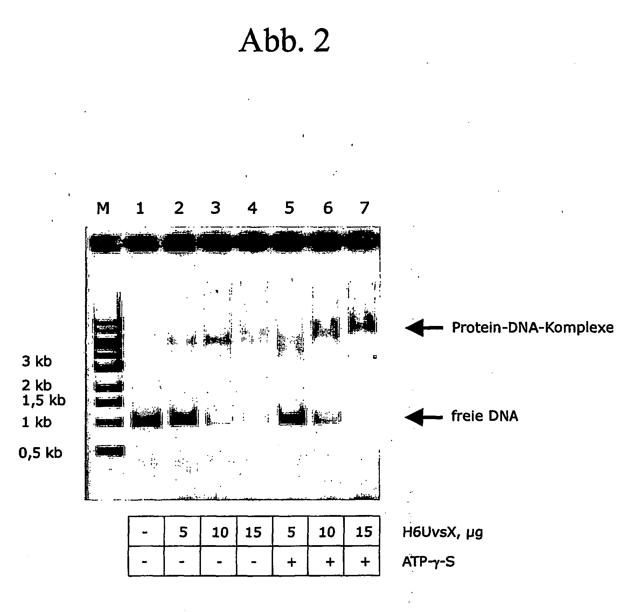

Abbildung 2 zeigt Ansätze von jeweils 200 ng eines gereinigten 1 kb-PCR-Fragments mit den dargestellten Mengen an H6UvsX in 76 mM K₂HPO₄, 17mM KH₂PO₄, 14mM NaH₂PO₄, pH=7,2, 5mM MgCl₂ und 0 bzw. 1 mM ATP-γ-S, die in einem Endvolumen von 15 µl für 30 min bei Raumtemperatur inkubiert und anschließend auf ein 0,8 %iges TAE/Agarose-Gel, das nachträglich mit Ethidiumbromid gefärbt wurde, aufgetragen wurden.

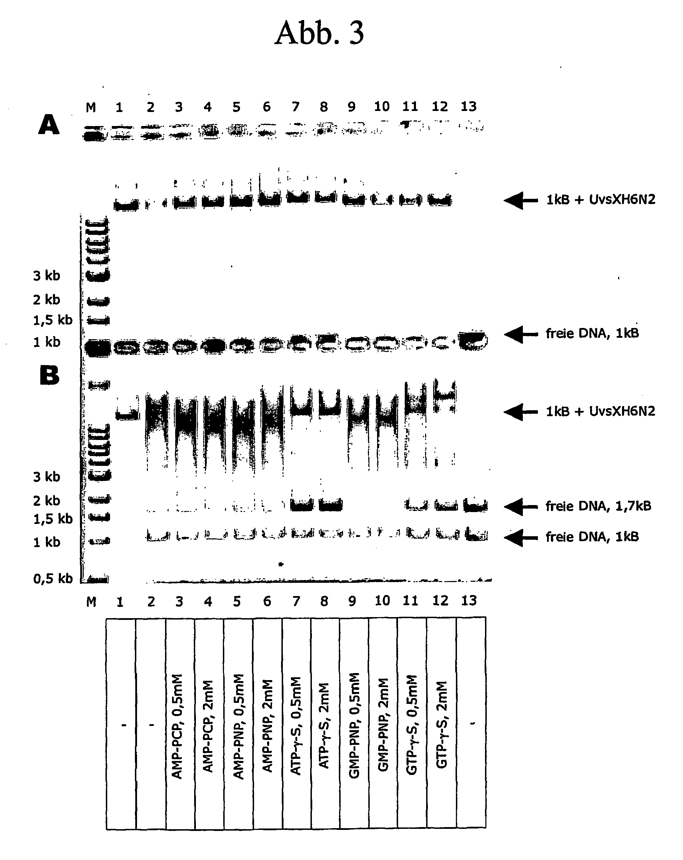

### Abbildung 3: Bindung von NLS-modifiziertem UvsX an doppelsträngige DNA in Gegenwart verschiedener ATP-Analoga

250 ng eines gereinigten 1 kb-PCR-Fragments wurden mit 15 µg UvsXH6N2 in 76 mM K₂HPO4, 17mM KH₂PO₄, 14mM NaH₂PO₄, pH=7,2, 5 mM MgCl₂ und 0,5 bzw. 2 mM der angegebenen Nukleotid-Analoga in einem Endvolumen von 20 µl für 30 min bei Raumtemperatur inkubiert. Anschließend wurden die Ansätze geteilt und zu einer Hälfte (B) 220 ng eines 1,7 kb-PCR-Fragmentes hinzugefügt, für weitere 30 min bei Raumtemperatur inkubiert und anschließend alle Ansätze auf ein 0,8%iges TAE/Agarose-Gel, das mit Ethidiumbromid nachträglich gefärbt wurde, aufgetragen.

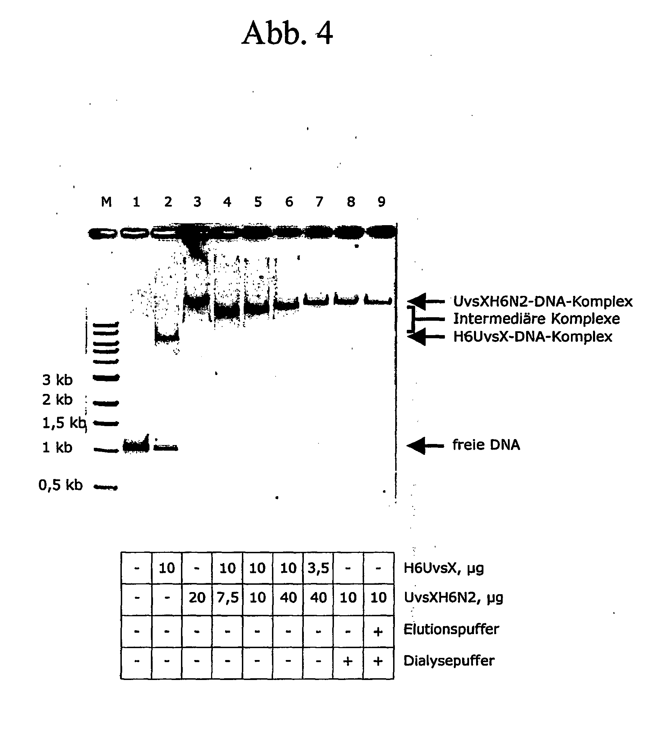

### Abbildung 4: Bindung einer Mischung aus UvsX und modifiziertem UvsX an doppelsträngige DNA

Jeweils 200 ng eines gereinigten 1 kb PCR-Fragments wurden in 76 mM K₂HPO₄, 17mM KH₂PO₄, 14mM NaH₂PO₄, pH=7,2, 5mM M₉Cl₂ und 1 mM ATP-γ-S mit den angegeben Mengen an gereinigtem H6UvsX bzw. UvsXH6N2 für 30 min bei RT inkubiert und anschließend alle Ansätze auf ein 0,8 %iges TAE/Agarose-Gel, das mit Ethidiumbromid nachträglich gefärbt wurde, aufgetragen. Um artifizielles Laufverhalten der DNA durch Salz, Imidazolspuren oder Glyzerin auszuschließen, wurde in Spalte 8 und 9 noch zusätzlich Elutions- bzw. Dialysepuffer hinzugefügt (Endkonzentration: 1/10 vol Elutionspuffer mit 500 mM Imidazol, 3/20 vol Dialysepuffer mit 50% Glyzerin).

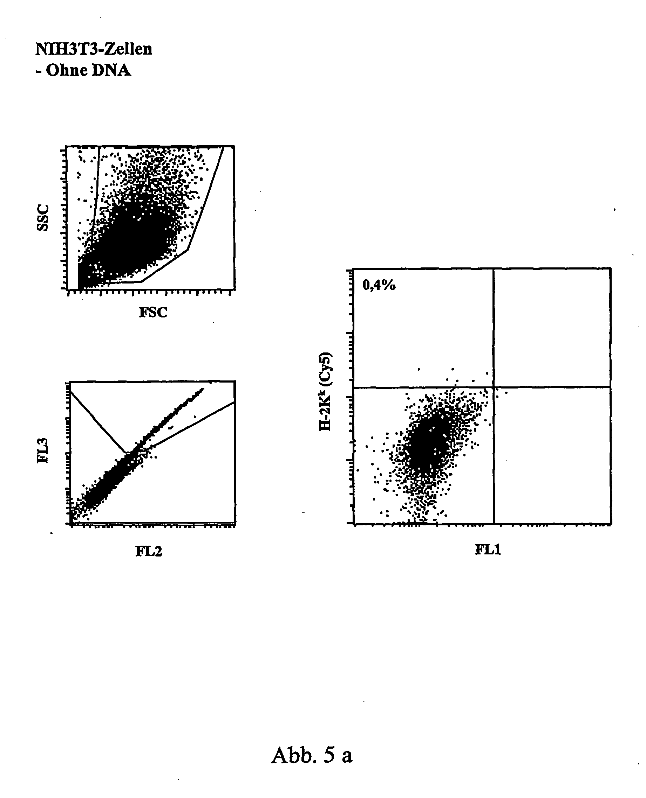

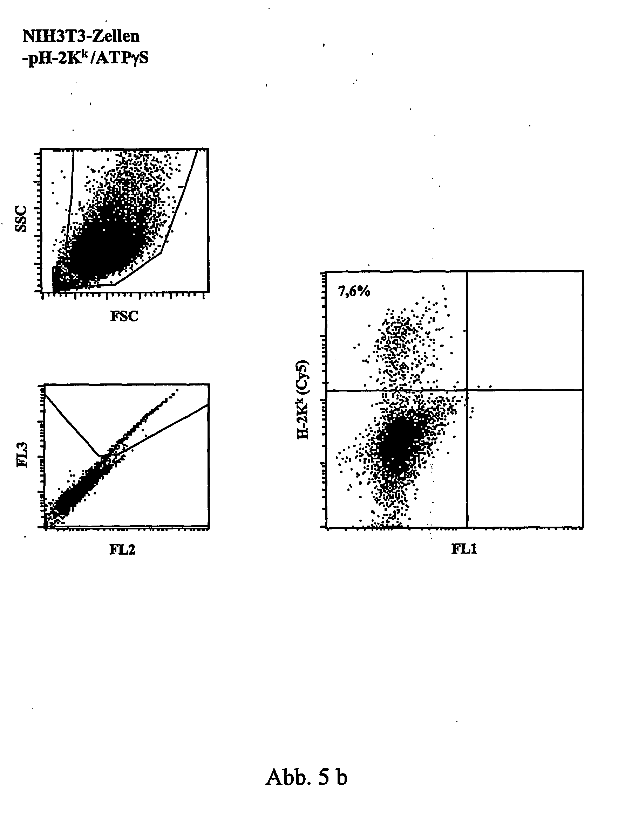

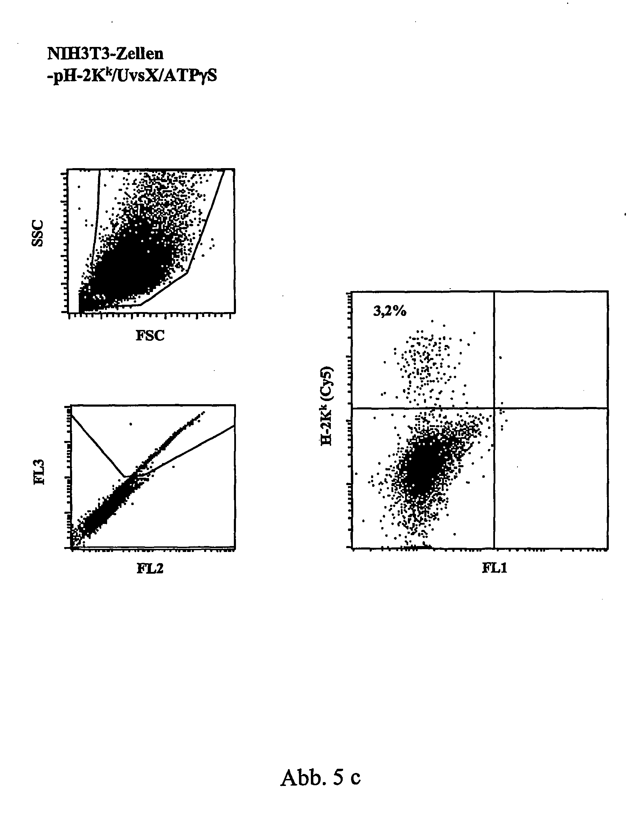

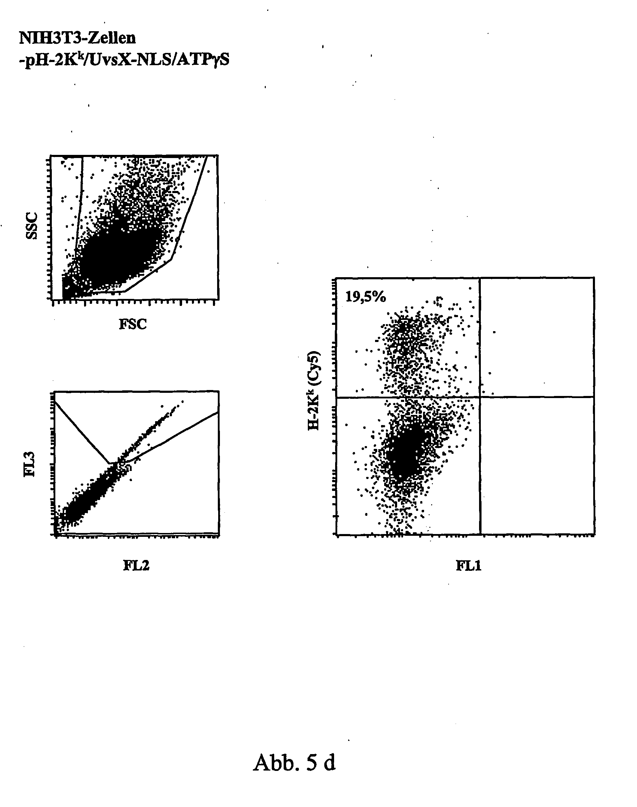

### Abbildung 5: FACScan-Analyse der Transfektion von NIH3T3-Zellen in Kombination mit Elektroporation

Abb. 5 a-d zeigt eine FACScan-Analyse: 5a) Elektroporation ohne DNA, 5b) Elektroporation von Vektor-DNA ohne UvsX, 5c) mit in UvsX verpackter Vektor-DNA, 5d) mit in UvsX-NLS verpackter Vektor-DNA.

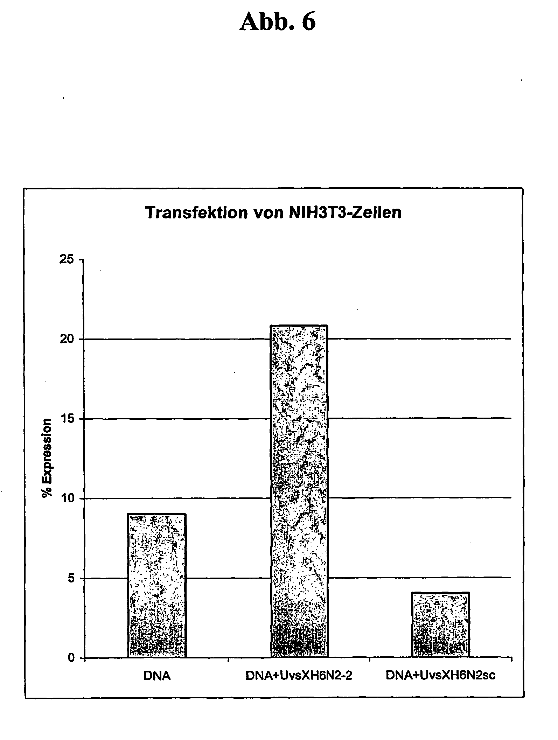

### Abbildung 6: Eine weitere FACScan-Analyse der Transfektion von NIH3T3-Zellen in Kombination mit Elektroporation

Abb. 6 zeigt ein Balkendiagramm der Ergebnisse einer FACScan-Analyse der Transfektion mit in UvsX-NLS (UvsXH6N2-2) verpackter Vektor-DNA und in UvsX-"scrambled"-NLS (UvsXH6N2sc) verpackter Vektor-DNA.

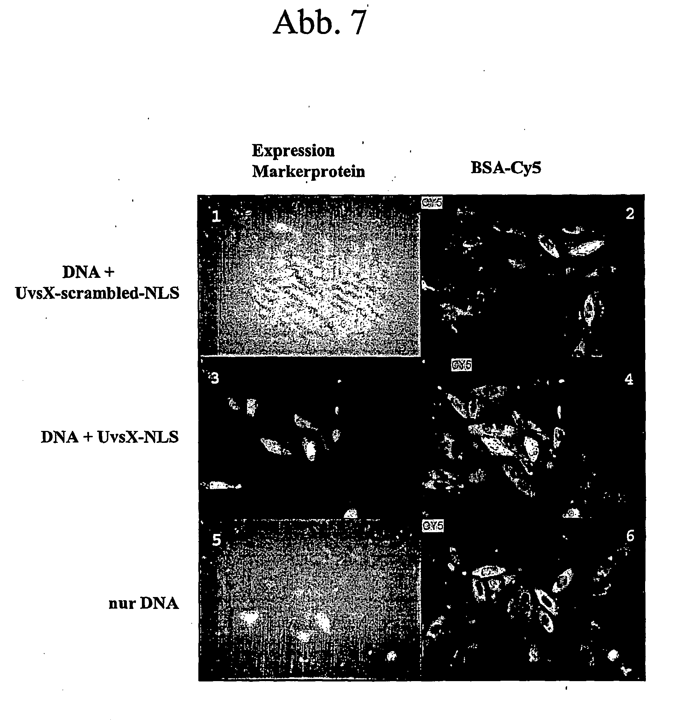

### Abbildung 7: Eine fluoreszenzmikroskopische Analyse der Transfektion von NIH3T3-Zellen in Kombination mit Mikroinjektion.

Gezeigt ist die Expression eines fluoreszierenden Markerproteins (linke Seite) in mikroinjizierten NIH3T3-Zellen. Als Injektionsmarker diente BSA-Cy5, das im entsprechenden Fluoreszenzfilter sichtbar gemacht wurde (rechte Seite). Die Bilder 1 und 2 zeigen Zellen, die mit DNA und UvsXH6N2sc ins Zytoplasma injiziert wurden, die Bilder 3 und 4 solche, die mit DNA und UvsXH6N2-2, die Bilder 5 und 6 solche, die nur mit DNA injiziert wurden.

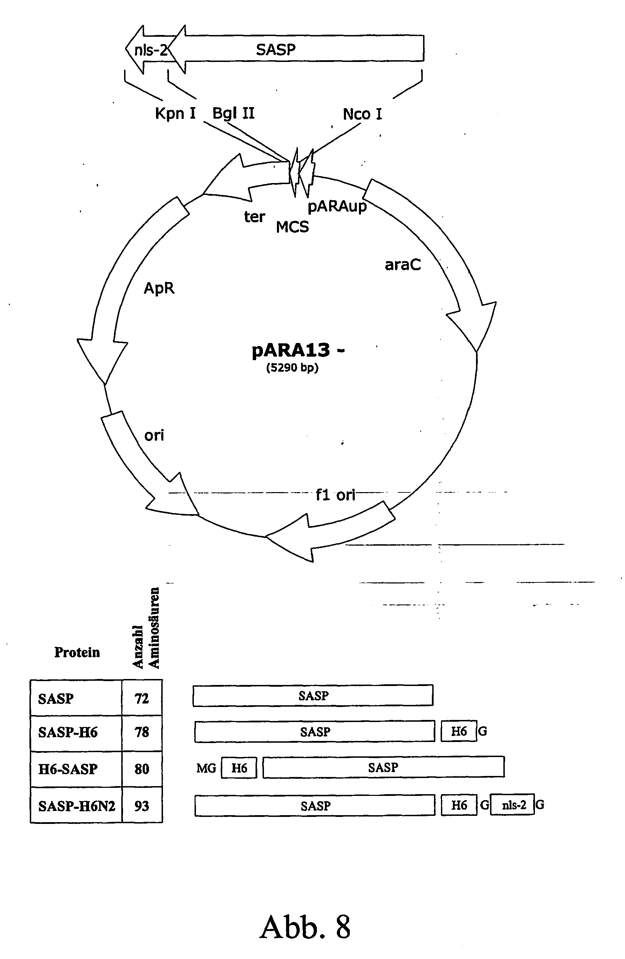

### Abbildung 8: Schematische Darstellung der Expressionsplasmide für SASP-Proteine

Abb. 8 zeigt eine schematische Darstellung des Aufbaus und der Herstellung der in den Beispielen 7 und 8 beschriebenen SASP-Proteine

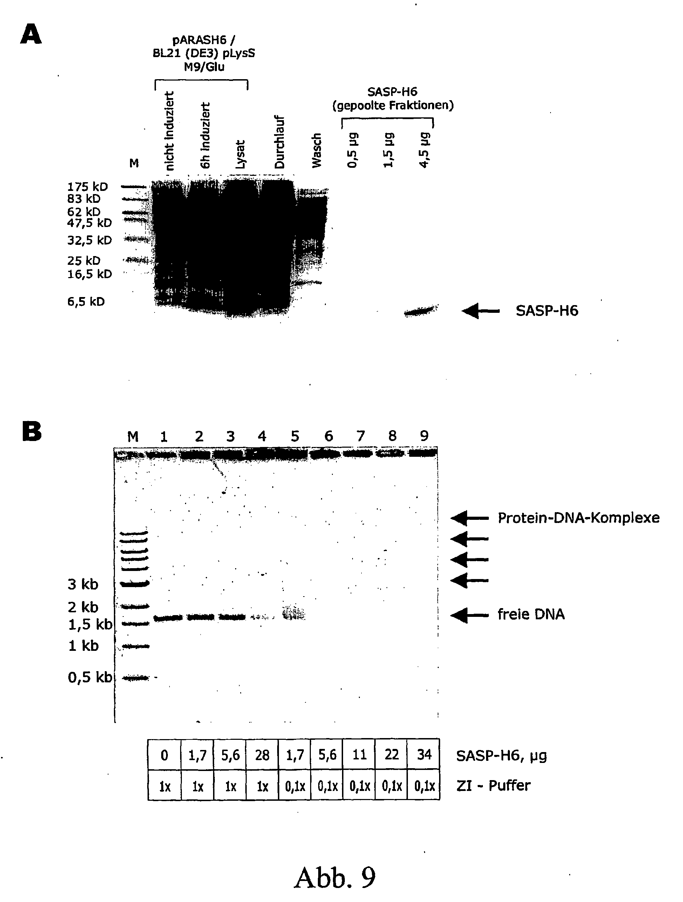

### Abbildung 9: (A) Aufreinigung von SASP-Protein (B) DNA-Bindungsfähigkeit von SASP-Protein

Abb. 9 A zeigt ein SDS-Gel mit gereinigtem SASP-Protein. Abb. 9 B zeigt die Bindung von DNA durch SASP-Protein in einer DNA-shift-Analyse.

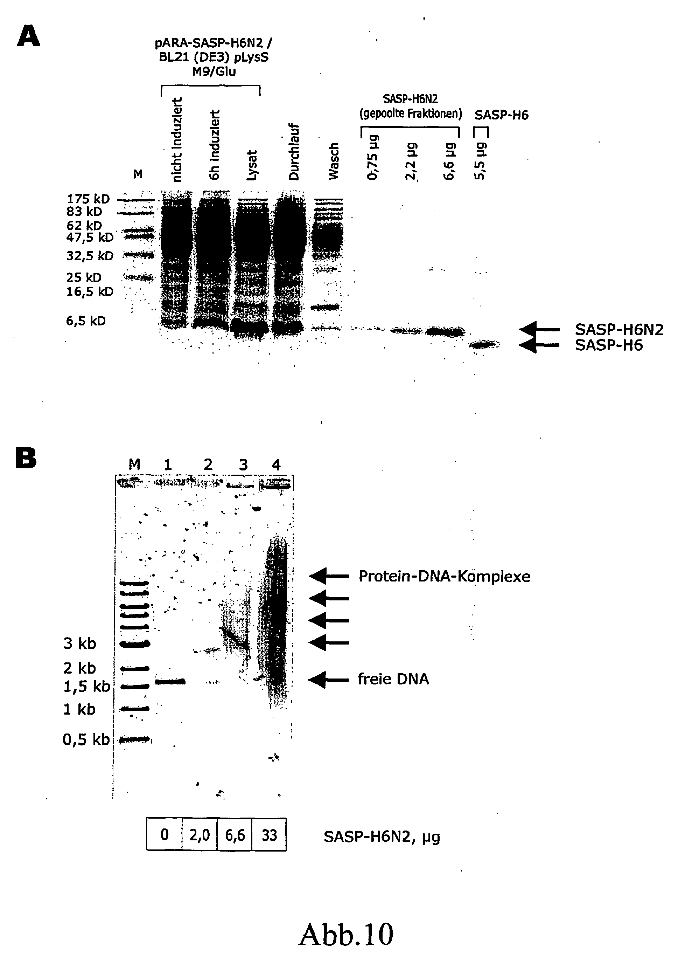

### Abbildung 10: (A) Aufreinigung von SASP-NLS-Protein (B) DNA-Bindungsfähigkeit des SASP-NLS-Proteins

Abb. 10 A zeigt ein SDS-Gel mit gereinigtem SASP-NLS-Protein (SASP-H6N2). Abb. 10 B zeigt die Bindung von DNA durch SASP-NLS-Protein in einer DNA-shift-Analyse.

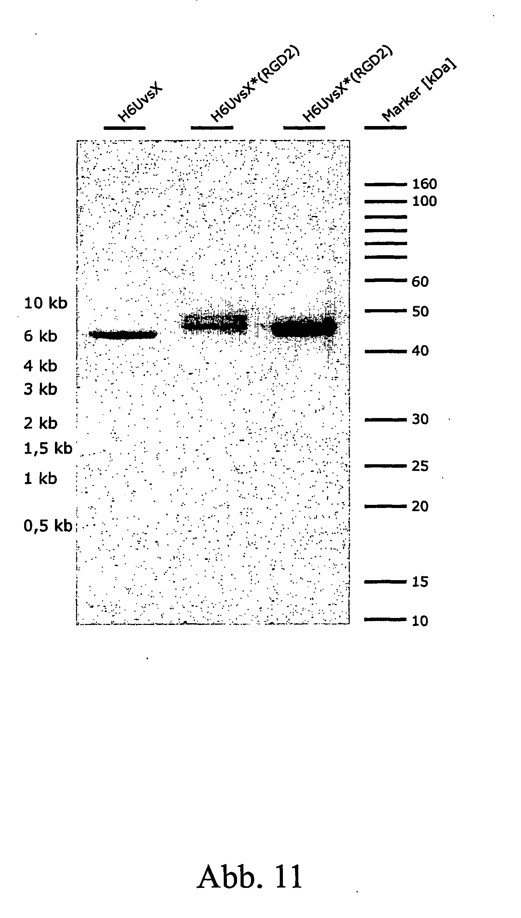

### Abbildung 11: Kopplung eines Peptids mit RGD-Motiv an H6UvsX

Abb. 11 zeigt die elektrophoretische Auftrennung von H6UvsX-Protein mit und ohne chemisch gekoppeltes Peptid RGD2. Gezeigt sind zwei unabhängig voneinander hergestellte Präparationen von H6UvsX*(RGD2). In jeder Spur wurde etwa 1 µg Protein eingesetzt.

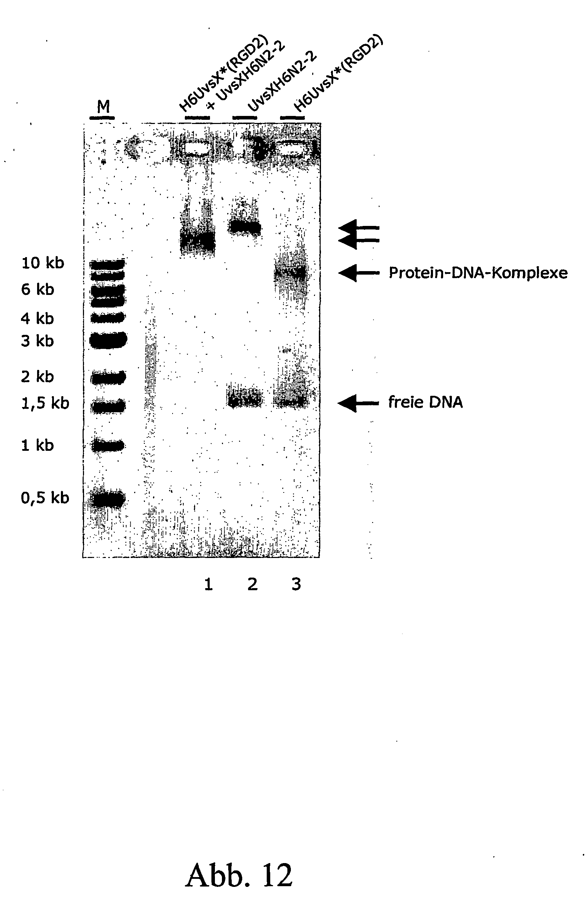

### Abbildung 12: Bindung von unterschiedlich modifizierten UvsX-Proteinen an doppelsträngige DNA

Abb. 12 zeigt in einem Agarose-Gel aufgetrennte NPFs bestehend aus einem doppelsträngigen DNA-Fragment und einem Gemisch aus H6UvsX*(RGD2) und UvsXH6N2-2 (Spur 1), oder aus UvsXH6N2-2 allein (Spur 2) oder aus H6UvsX*(RGD2) allein (Spur 3).

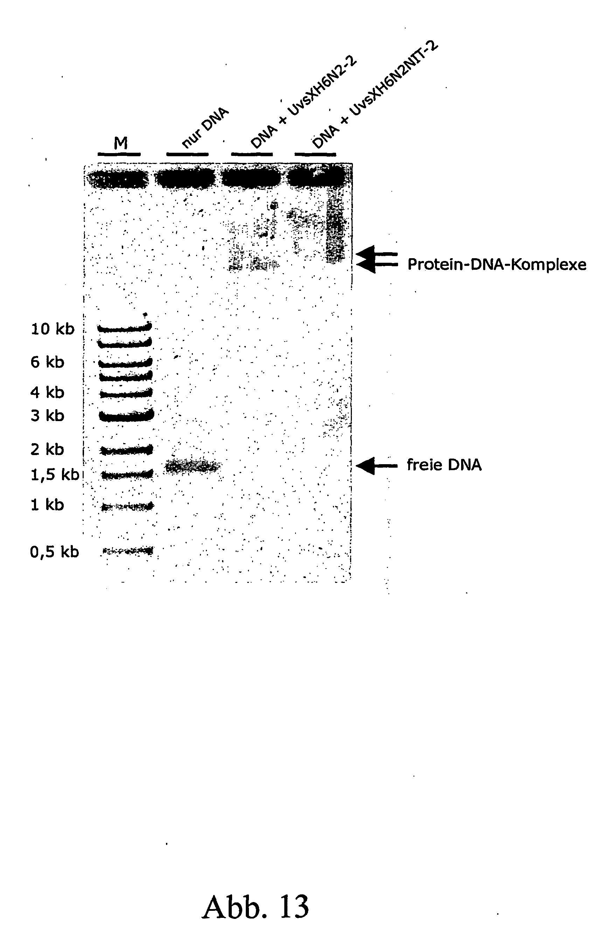

### Abbildung 13: DNA-Shift-Analyse der Bindung von UvsXH6N2 und UvsXH6N2NIT-2 an Fluoreszein-markierte DNA

Abb. 13 zeigt einen DNA-Shift von DNA-UvsXH6N2 und DNA-UvsXH6N2NIT-2.
Die Proteine wurden mit einem AlexaFluor488-marklerten DNA-Fragment inkubiert wie in Beispiel 10 beschrieben.

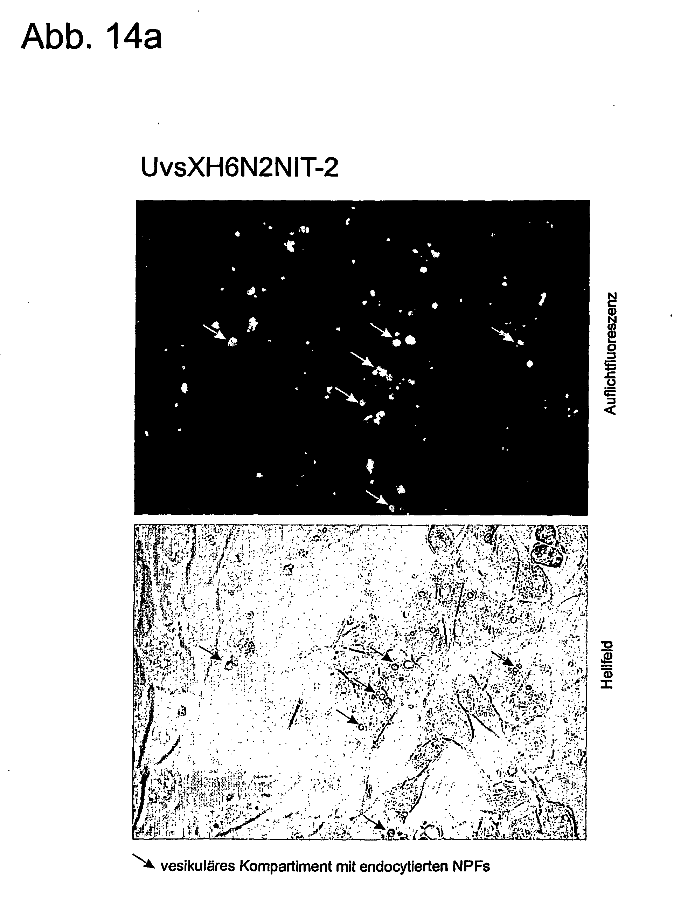

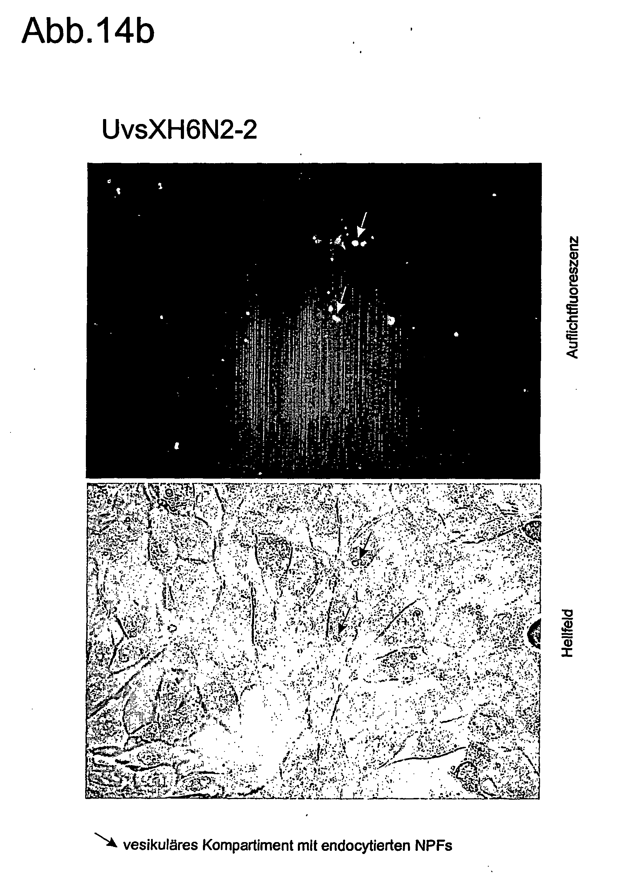

### Abbildung 14: Fluoreszenzmikroskopische Analyse der Aufnahme von NPFs in NIH3T3-Zellen durch Endozytose

Abb. 14a und 14b zeigen fluoreszenzmikroskopische Bilder von NIH3T3-Zellen. Gezeigt sind jeweils eine Aufnahme im Hellfeld (unten) und eine in Auflichtfluoreszenz (oben). Zu erkennen sind im Hellfeld vesikuläre intrazelluläre Kompartimente, die im Fluoreszenzlicht aufgrund der endozytierten, Fluoreszeinmarkierten DNA aufleuchten.

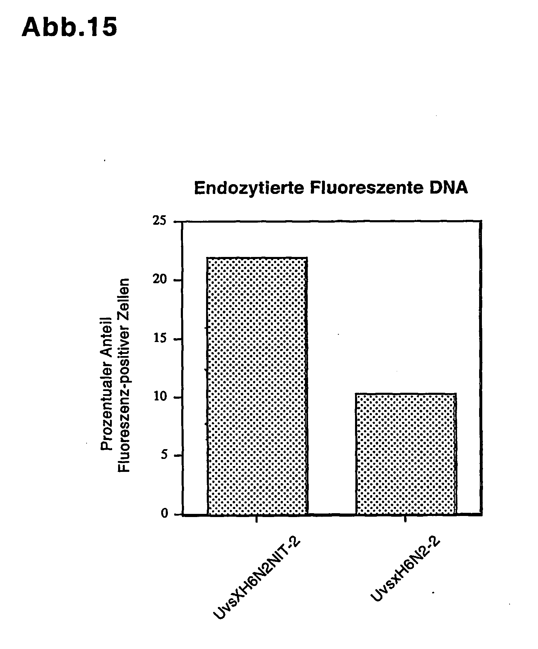

### Abbildung 15: Prozentualer Anteil von Zellen mit endocytierten NPFs

Abb. 15 zeigt in einem Balkendiagramm den prozentualen Anteil der Zellen, die mindestens ein fluoreszierendes vesikuläres Kompartiment besitzen.

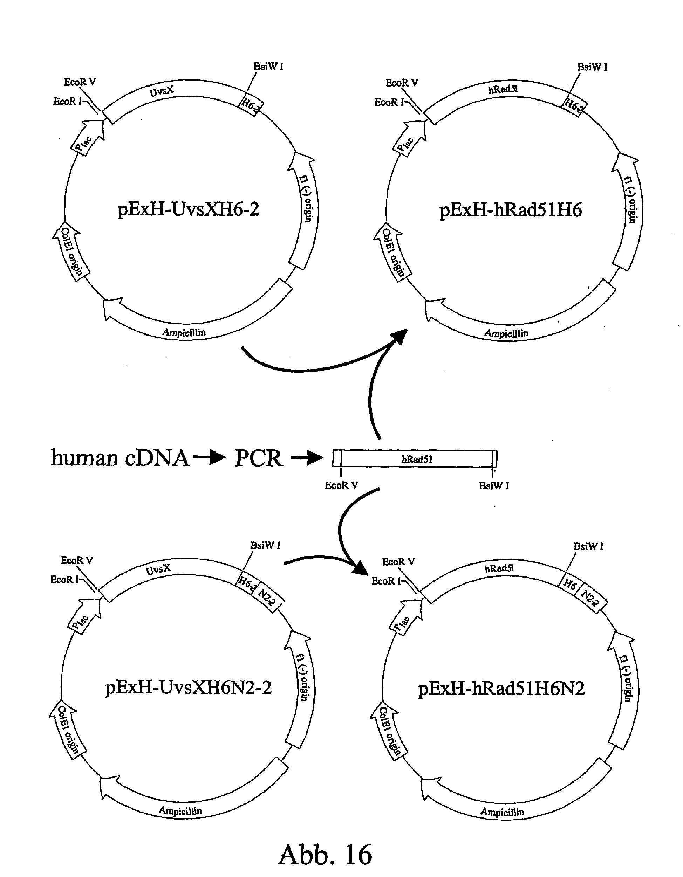

### Abbildung 16: Schematische Darstellung der Expressionsplasmide

Abb. 16 zeigt die schematische Darstellung des Aufbaus der Expressionsplasmide zur Herstellung von hRad51-Fusionsproteinen.

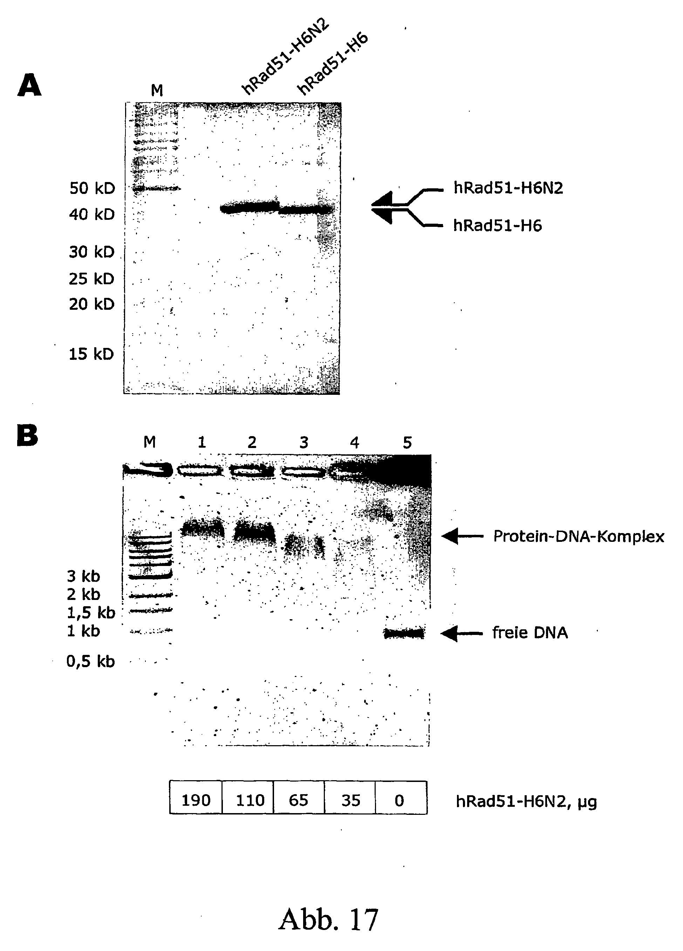

### Abbildung 17: Bindung von NLS-modifiziertem hRad51 (hRad51H6N2) an doppelsträngige DNA

Abb. 17 (A) zeigt ein SDS/Coomassie-Gel mit hRad51H6N2 (12,7 µg) und hRad51H6 (11 µg) nach der Reinigung über Nickel-Chelat-Affinitätschromatografie. Abb. 17 (B) zeigt Ansätze von jeweils 100 ng eines gereinigten 0,9 kb-PCR-Fragments mit den dargestellten Mengen an hRad51H6N2 in 38 mM K₂HPO₄, 8,5 mM KH₂PO₄, 7 mM NaH₂PO₄, pH=7,2, 15 mM MgCl₂, 2,5 mM ATP und 25% Glycerin, die in einem Endvolumen von 30 µl für 10 min bei 37°C inkubiert und anschließend auf ein 1 %iges TAE/Agarose-Gel, das nachträglich mit Ethidiumbromid gefärbt wurde, aufgetragen wurden.

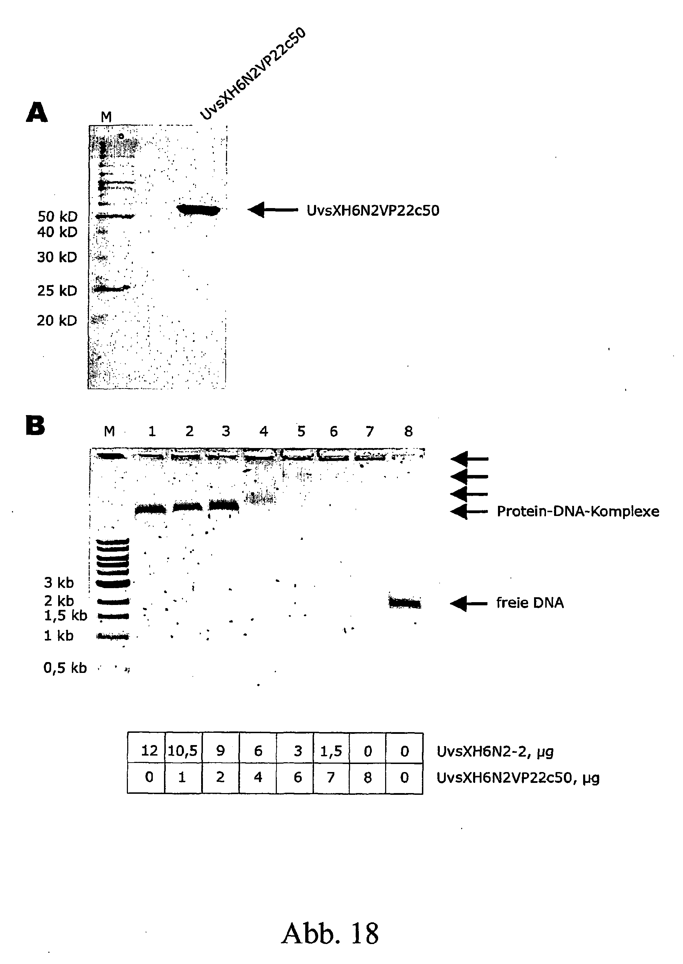

### Abbildung 18 Bindung einer Mischung aus verschieden modifizierten UvsX (UvsX-NLS-VP22 und UvsX-NLS) an doppelsträngige DNA

Abb. 18 (A) zeigt ein SDS/Coomassie-Gel mit UvsXH6N2VP22c50.
Abb. 18 (B): Jeweils 140 ng eines gereinigten 1,7 kb PCR-Fragments wurden in 96 mM K₂HPO₄, 21,5 mM KH₂PO₄, 18mM NaH₂PO₄, pH=7,2, 5mM MgCl₂ und 1,3 mM ATP-γ-S mit den angegeben Mengen an gereinigtem UvsXH6N2VP22c50 bzw. UvsXH6N2-2 für 30 min bei RT inkubiert und anschließend alle Ansätze auf ein 0,8 %iges TAE/Agarose-Gel, das mit Ethidiumbromid nachträglich gefärbt wurde, aufgetragen.

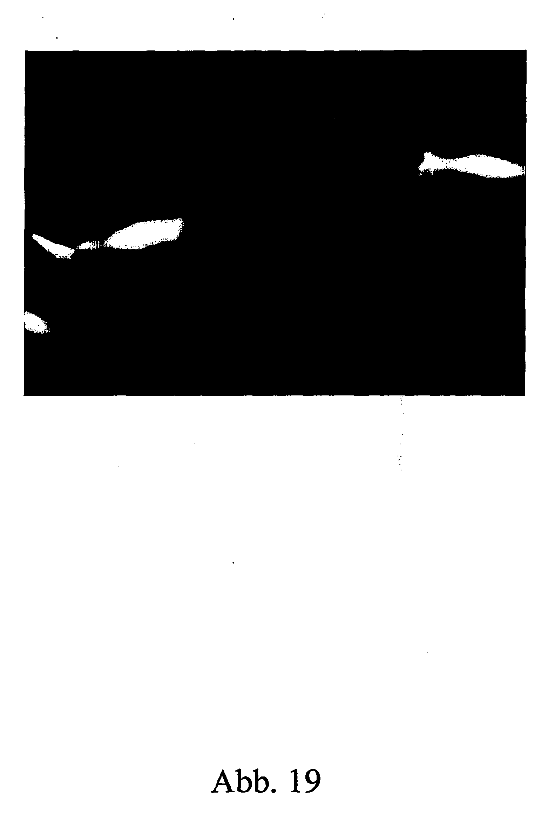

### Abbildung 19: Transfizierte NIH3T3 Zellen nach Behandlung mit Komplexen aus DNA und einer Mischung aus UvsX-NLS-VP22 und UvsX-NLS

Fluoreszenzmikroskopische Aufnahme von NIH3T3 Zellen, 24 h nach der Behandlung mit Komplexen aus 1 µg Expressionsvektor-DNA und einer Mischung aus 19 µg UvsXH6N2VP22c50 und 39 µg UvsXH6N2-2.

### Abbindung 20: Schematische Darstellung des prinzipiellen Aufbaus des erfindungsgemäßen Verfahrens und Transfektionsagens basierend auf Nukleoproteinfilamenten

### Beschreibung der Erfindung

Die nachfolgenden Beispiele dienen der näheren Erläuterung der Erfindung, ohne diese jedoch auf die beispielhaft offenbarten Stoffe und Verfahren zu beschränken.

### Beispiel 1: Herstellung von rekombinanten UvsX als NPF-bildende Proteine

Als NPF-bildende Proteine wurden die Proteine UvsXH6, UvsXH6-2, H6UvsX und H6UvsX-2 verwendet (siehe. Abb. 1).

### Aufbau der Proteine:

### UvsXH6 (400 Aminosäuren):

Aminosäuren 1-391: UvsX aus dem Phagen T4 (NCBI-Protein Accession-No.: AAD42669, Aminosäuren 1-391), Aminosäuren 392-394: Linker aus den Aminosäuren G³⁹²GS³⁹⁴, Aminosäuren 395-400: H³⁹⁵HHHHH⁴⁰⁰ für die Aufreinigung über Nickel-Chelat-Affinitätschromatografie, Aminosäureaustausch: L⁴³→P.

### UvsXH6-2 (403 Aminosäuren):

Aminosäuren 1-391: UvsX aus dem Phagen T4 (NCBI-Protein Accession-No.: AAD42669, Aminosäuren 1-391), Aminosäuren 392-394: Linker aus den Aminosäuren S³⁹²YG³⁹⁴, Aminosäuren 395-400: H³⁹⁵HHHHH⁴⁰⁰, Aminosäuren 401-403: C-Terminus aus den Aminosäuren M⁴⁰¹YS⁴⁰³,

### H6UvsX (404 Aminosäuren):

Aminosäuren 1-4: N-Terminus aus den Aminosäuren M¹SYS⁴, Aminosäuren 5-10: H⁵HHHHH¹⁰, Aminosäuren 11-13: Linker aus den Aminosäuren S¹¹YG¹³, Aminosäuren 14-404: UvsX aus dem Phagen T4 (NCBI-Protein Accession-No.: AAD42669, Aminosäuren 1-391), Aminosäureaustausch: Q³⁴⁰→L.

### H6UvsX-2 (404 Aminosäuren):

Aminosäuren 1-4: N-Terminus aus den Aminosäuren M¹GYS⁴, Aminosäuren 5-10: H⁵HHHHH¹⁰, Aminosäuren 11-13: Linker aus den Aminosäuren S¹¹YG¹³, Aminosäuren 14-404: UvsX aus dem Phagen T4 (NCBI-Protein Accession-No.: AAD42689, Aminosäuren 1-391).

### Klonierung der Expressionsplasmide

Für die Expression der oben genannten Proteine in geeigneten *Escherichia coli* Zellen wurden Plasmide konstruiert, die eine kodierende Sequenz für UvsXH6, UvsXH6-2, H6UvsX bzw. H6UvsX-2 unter der Kontrolle des lac-Promoters enthalten (pExH-UvsXH6, pExH-UvsXH6-2, pExH-H6UvsX bzw. pExH-H6UvsX-2, siehe Abb. 1). Die Plasmide wurden durch Ligation zweier PCR-Produkte hergestellt. Das erste PCR-Produkt wurde von pMCS5 (MoBiTec, Göttingen, Deutschland) amplifiziert. pMCS5 ist ähnlich aufgebaut wie pBluescript SK(-) (Stratagene) und unterscheidet sich von diesem nur im 5'-Bereich der kodierenden Sequenz für das lacZα-Fragment. pMCS5 besitzt daher den lac-Promoter und nachgeschaltet den lac-Operator, wodurch die Expression einer eingefügten kodierenden Sequenz von der Abwesenheit von aktivem lac-Repressor abhängt. Um in jedem Fall eine konstitutive Expression zu erreichen, wurden die Amplifikationsprimer so gewählt, dass der lac-Operator im PCR-Produkt nicht mehr enthalten ist. Das resultierende PCR-Produkt entsprach pMCS5 von Position 992 bis 664 zuzüglich Restriktionsüberhängen. Vor der Position 992 (3' zum lac-Promoter) wurden die Nukleotide GAATTC (EcoR I Restriktionsschnittstelle) sowie TGTGTG und hinter der Position 664 die Nukleotide ACTAGT (Spe I Restriktionsschnittstelle) und CACACA angefügt, um die Ligation nach Restriktionsverdau mit EcoR I und Spe I zu ermöglichen. Die kodierende Sequenz für UvsXH6, UvsXH6-2 bzw. H6UvsX und H6UvsX-2 wurde durch PCR-Amplifikation von T4-DNA mit Primern gewonnen, die die gewünschten Restriktionsschnittstellen, eine Ribosomenbindungsstelle und die zusätzlichen Codons enthalten. Die PCR-Produkte enthielten am 5'-Ende vor dem Startcodon die zusätzlichen Nukleotide 5'-CACACAGAATTCATAAAGGAAGATATCAT-3', sowie am 3'-Ende nach dem Stopcodon die zusätzlichen Nukleotide 5'-ACTAGTTGTGTG-3'.

### Reinigung:

1,5-3 I dYT/Ampicillin(200µg/ml) wurden 1:1000 mit einer Übernachtkultur von pExH-H6UvsX in DH5 angeimpft und über Nacht bei 37° C mit 250 UPM wachsen gelassen. Die bei 7000 x g geernteten Kulturen ergaben ca. 7-15 g Bakteriensediment. Dieses wurde für 1-3 Tage bei -20° C eingefroren. Das Sediment wurde auf Eis getaut und in 10-20 ml kaltem Start-Puffer resuspendiert. Die Lyse fand mit Hilfe von 10 mg Lysozym (Serva, 190.000 u/mg) und ca. 4 g Glaskügelchen (Sigma, G-8893) unter langsamem Rühren für 1 h bei 4° C statt. Anschließend wurden 50 µl DNAse I (Serva, 2 mg/ml) hinzugefügt und für weitere 30 min inkubiert. Nach Abzentrifugieren des Lysats (45 min 11000 x g, 4 °C) wurde der Überstand durch einen Sterilfilter (Porengröße 0,45 µm) filtriert und auf eine mit Ni⁺⁺-lonen beladene und äquilibrierte 1 ml HiTrapTM Chelating-Säule (Pharmacia) geladen. Die weiteren Aufreinigungsschritte erfolgten nach dem entsprechenden Pharmacia-Protokoll für Proteine, die mit einem Histidin-Hexamer versehen wurden. Aliquots der verschiedenen Elutionsfraktionen wurden auf SDS/Coomassie-Gelen aufgetragen. Die jeweils saubersten Fraktionen wurden vereinigt und über Centriplus YM30-Säulen (Millipore) nach dem entsprechenden Protokoll weiter eingeengt. Danach erfolgte eine zweimalige Dialyse (Dialyseschlauch: Spectra/Por, MWCO: 25.000) gegen ein mindestens eintausendfaches Volumen ZI-Puffer für jeweils mindestens eine Stunde bei 4° C, dann über Nacht bei 4° C gegen ZI-Puffer/50 % Glyzerin. Das Dialysat wurde in 30-50 µl-Fraktionen aliquotiert und bei -80° C aufbewahrt.

### Verwendete Puffer:

ZI-Puffer: 76 mM K₂HPO₄, 17 mM KH₂PO₄, 14 mM NaH₂PO₄, pH=7,2
Startpuffer: 20 mM Pi, 0,5 M NaCl, 10 mM Imidazol, pH=7,4
Waschpuffer: 20 mM Pi, 0,5 M NaCl, 20-50 mM Imidazol, pH=7,4
Elutionspuffer: 20 mM Pi, 0,5 M NaCl, 100-500 mM Imidazol, pH=7,4

### Konzentrationsbestimmung:

Die Konzentration der UvsX-Proteine wurde durch Messen der OD₂₈₀ unter Einbeziehung des über das Programm Gene Inspector^{™}(Textco, Inc.) errechneten Extinktionskoeffizienten bestimmt. Bei H6UvsX betrug sie 2,5-3,5 µg/µl.

### Versuchsbeschreibung:

Über Ni⁺⁺-Sepharose gereinigtes H6UvsX wurde mit jeweils 200 ng eines 1 kb PCR-Fragmentes (mit und ohne 1 mM ATP-γ-S) inkubiert.
Ein durch Proteinbindung verursachter Shift der DNA im Agarosegel zeigt, dass H6UvsX in Abhängigkeit von der Konzentration, doppelsträngige DNA bindet, und dass diese Bindung durch ATP-γ-S verstärkt wird.
Mit ATP-γ-S entstehen Protein-DNA-Komplexe, die mit Ethidiumbromid stärker angefärbt werden als ohne ATP-γ-S, was einen Hinweis auf eine topologische Veränderung des Nucleoproteinfilamentes durch das Nukleotidanalogon darstellt.

### Beispiel 2: Herstellung eines Transfektionsagens, basierend auf UvsX als NPF-bildendem Protein mit einem Kernlokalisationssignal als funktioneller Komponente

In Analogie zu Beispiel 1 wurden Plasmide hergestellt, die die Expression der Fusionsproteine UvsXH6N2, UvsXH6N2-2, N2H6UvsX und N2H6UvsX-2 erlauben, die ausgehend von den im Beispiel 1 beschriebenen Proteinen zusätzlich ein Kernlokalisationssignal enthalten (siehe Abb. 1).

### Aufbau der Proteine:

### UvsXH6N2 (426 Aminosäuren):

Aminosäuren 1-391: UvsX aus dem Phagen T4 (NCBI-Protein Accession-No.: AAD42669, Aminosäuren 1-391), Aminosäuren 392-394: Linker aus den Aminosäuren G³⁹²GS³⁹⁴, Aminosäuren 395-400: H³⁹⁵HHHHH⁴⁰⁰, Aminosäuren 401-403: Linker aus den Aminosäuren G⁴⁰¹GS⁴⁰³, Aminosäuren 404-417: Kernlokalisationssignal nls-2 (Aminosäuren 2-15, SEQ ID NO: 9 aus WO 00/40742), Aminosäuren 418-421: C-Terminus von UvsX aus dem Phagen T4 (NCBI-Protein Accession-No.: AAD42669, Aminosäuren 388-391), Aminosäuren 422-426: C-Terminus aus den Aminosäuren K⁴²²LVTG⁴²⁶, Aminosäureaustausch: Y²³⁸→V.

### UvsXH6N2-2 (420 Aminosäuren):

Aminosäuren 1-391: UvsX aus dem Phagen T4 (NCBI-Protein Accession-no.: AAD42669, Aminosäuren 1-391), Aminosäuren 392-394: Linker aus den Aminosäuren S³⁹²YG³⁹⁴, Aminosäuren 395-400: H³⁹⁵HHHHH⁴⁰⁰, Aminosäuren 401-403: Linker aus den Aminosäuren M⁴⁰¹YS⁴⁰³, Aminosäuren 404-417: Kernlokalisationssignal nls-2 (Aminosäuren 2-15, SEQ ID NO: 9 aus WO 00/40742), Aminosäuren 418-420: C-Terminus aus den Aminosäuren G⁴¹⁸YP⁴²⁰.

### N2H6UvsX (420 Aminosäuren):

Aminosäuren 1-3: N-Terminus aus den Aminosäuren M¹SY³, Aminosäuren 4-17: Kernlokalisationssignal nls-2 (Aminosäuren 2-15, SEQ ID NO: 9 aus WO 00/40742), Aminosäuren 18-20: Linker aus den Aminosäuren L¹⁸YS²⁰, Aminosäuren 21-26: H²¹HHHHH²⁶, Aminosäuren 27-29: Linker aus den Aminosäuren S²⁷YG²⁹, Aminosäuren 30-420: UvsX aus dem Phagen T4 (NCBI-Protein Accession-No.: AAD42669, Aminosäuren 1-391), Aminosäureaustausch: Q³⁵⁶→L.

### N2H6UvsX-2 (421 Aminosäuren):

Aminosäuren 1-4: N-Terminus aus den Aminosäuren M¹GYP⁴, Aminosäuren 5-18: Kernlokalisationssignal nls-2 (Aminosäuren 2-15, SEQ ID NO: 9 aus WO 00/40742), Aminosäuren 19-21: Linker aus den Aminosäuren S¹⁹YS²¹, Aminosäuren 22-27: H²²HHHHH²⁷, Aminosäuren 28-30: Linker aus den Aminosäuren S²⁸YG³⁰, Aminosäuren 31-421: UvsX aus dem Phagen T4 (NCBI-Protein Accession-No.: AAD42669, Aminosäuren 1-391).

### Klonierung der Expressionsplasmide

Für die Expression in geeigneten *Escherichia coli* Zellen wurden Plasmide konstruiert, die eine kodierende Sequenz für UvsXH6N2, UvsXH6N2-2, N2H6UvsX bzw. N2H6UvsX-2 unter der Kontrolle des lac-Promoters enthalten (pExH-UvsXH6N2, pExH-UvsXH6N2-2, pExH-N2H6UvsX bzw. pExH-N2H6UvsX-2, s. Abb. 1). Die Plasmide wurden wie im Beispiel 1 beschrieben durch Ligation zweier PCR-Produkte hergestellt (s. Abbildung 1).

### Reinigung:

Die Reinigung von UvsXH6N2 erfolgte wie unter Beispiel 1) für H6UvsX beschrieben. Konzentration: 10-20 µg/µl.

### Versuchsbeschreibung:

UvsXH6N2 bindet an doppelsträngige DNA. Die Bindung wird durch ATP-γ-S stabilisiert:
Um den Einfluß verschiedener ATP-Analoga auf das Bindungsverhalten von UvsXH6N2 an DNA zu untersuchen, wurde das Protein zunächst mit einem 1 kb-DNA-Fragment und verschiedenen ATP-Analoga inkubiert. Anschließend wurde zur Kompetition ein 1,7 kb großes DNA-Fragment zugegeben. Erfolgt durch Zugabe eines ATP-Analogon eine Stabilisierung der Bindung des Proteins an die DNA, so ist zu erwarten, dass ein kompetierendes DNA-Fragment mit geringerer Wahrscheinlichkeit von UvsXH6N2 gebunden wird, solange sich noch kein Gleichgewicht eingestellt hat. Wie in Abb. 3 zu erkennen, bleibt der Protein-DNA-Komplex, der mit dem 1 kb großen Fragment und UvsXH6N2 entsteht, in Anwesenheit von ATP-γ-S und dem 1,7 kb-Fragment im Vergleich zu allen anderen benutzten ATP-Analoga stabil, d. h. das 1,7 kb-Fragment wird offenbar nicht oder nur marginal innerhalb der beobachteten Zeit von freigewordenen UvsXH6N2-Molekülen besetzt.

### Beispiel 3: Herstellung eines Transfektionsagens mit einer Mischung aus modifiziertem und nicht-modifiziertem NPF-bildendem Protein

### Versuchsbeschreibung:

Verschiedene Verhältnisse von H6UvsX und UvsxH6N2 wurden mit einem 1 kb DNA-Fragment inkubiert. Die zwei Proteine retardieren aufgrund ihres unterschiedlichen Molekulargewichts die DNA unterschiedlich stark (siehe Spalte 2 und 3 von Abbildung 4). Mischt man die Proteine vor der Zugabe zur DNA, so entstehen je nach Verhältnis von H6UvsX zu UvsXH6N2 intermediäre Komplexe, die wiederum eine scharfe Bande ergeben und somit ein gleiches durchschnittliches Molekulargewicht haben. Dies zeigt, dass die DNA statistisch gleichmäßig mit beiden Proteinen belegt ist.

### Beispiel 4: Transfektion einer Zelllinie (NIH3T3) mit UvsX-NLS in Kombination mit Elektroporation

NIH3T3-Zellen (adhärent, bis zur 70 - 80 %igen Konfluenz kultiviert) wurden mit einem Vektor, der für die schwere Kette des Maus MHC Klasse I Proteins H-2K^{K} kodiert, transfiziert. 1 x 10⁶ Zellen wurden mit 25 ng Vektor-DNA, die zuvor in Bindungspuffer (76 mM K₂HPO₄, 17 mM KH₂PO₄, 14 mM NaH₂PO₄, 5 mM MgCl₂ pH 7,21) mit 14 µg UvsX bzw. UvsX-NLS sowie mit oder ohne 1 mM ATP-γ-S für 30 Minuten bei Raumtemperatur vorinkubiert wurde, elektroporiert. Dazu wurden die Zellen in Elektroporationspuffer (103 mM NaCl, 5,36 mM KCl, 0,41 mM MgCl₂, 23,8 mM NaHCO₃, 5,64 mM Na₂HPO₄, 11,2 mM Glucose, 0,42 mM Ca(NO₃)₂, 20 mM HEPES, 3,25 µM Gluthathion) in einem Gesamtvolumen von 100 µl aufgenommen und in einer Küvette mit 2 mm Elektrodenabstand elektroporiert. Die Elektroporation erfolgte über eine exponentielle Entladung bei einer Spannung von 240 V und einer Kapazität von 450 µF. Die Halbwertzeit des Spannungsabfalls betrug typischerweise 12 msec. Unmittelbar nach der Elektroporation wurden die Zellen mit Kulturmedium (RPMI mit 10 % FCS) aus den Küvetten gespült, für 10 min bei 37 °C inkubiert und dann in einer Kulturschale mit vorgewärmtem Kulturmedium überführt. Nach 6 h Inkubation wurden die Zellen geerntet und nach zweimaligem Waschen in PBS mit einem Cy5-gekoppelten-anti-H-2K^{K}-Antikörper inkubiert und durchflußzytometrisch (FACScan) analysiert. Durch Färbung mit Propidiumjodid wurde die Anzahl toter Zellen bestimmt. Sechs Stunden nach der Elektroporation exprimieren (abzüglich des Hintergrundes von durchschnittlich 0,25%) 7,4% bzw. 8,7% der mit freier Vektor-DNA transfizierten Zellen das H-2K^{K}-Protein. Im Vergleich dazu liegt die Expressionsrate der Zellen, die mit Vektor-UvsX transfiziert wurden bei 2,9% bzw. 3,8%. Die Expressionsrate bei einer Transfektion mit Vektor-UvsX-NLS beträgt 19,2% bzw. 18,9% (siehe Abbildungen 5a-5d).

Zur Untersuchung des Kerntransportes wurde das physikalische Verfahren der Elektroporation gewählt, damit außer UvsX keine weiteren biochemischen Komponenten einen Einfluss auf die Transfektion haben. Die feste Bindung von UvsX mit ATP-γ-S an DNA beeinträchtigt allerdings die Beweglichkeit des Komplexes im elektrischen Feld und reduziert damit die Effizienz der Elektroporation um ca. 60%. Allein die Anbringung eines Kerntransportsignals führt in diesem System zu einer Steigerung der Expression kurz nach der Transfektion um einen Faktor von durchschnittlich 5,7. Die Steigerung der Expressionsrate durch UvsX-NLS im Vergleich zu UvsX zeigt also, dass bei Verwendung eines Kemtransportsignals als funktioneller Komponente DNA durch UvsX in den Kern befördert wird. Eine Analyse bereits kurze Zeit nach der Transfektion wird erheblich dadurch verbessert, dass auch solche Zellen zugänglich werden, die sich zwischen Transfektion und Analyse nicht geteilt haben.

### Beispiel 5: Transfektion einer Zelllinie (NIH3T3) mit UvsX-scrambled-NLS bzw. UvsX-NLS in Kombination mit Elektroporation

### Herstellung von UvsX-scrambled-NLS

Um den Einfluss des Kerntranslokalisationssignals auf die Transfektion testen zu können, wurde für Vergleichszwecke ein UvsX-Derivat hergestellt, das in seiner Nettoladung einem UvsX-Protein mit einer NLS entspricht, selbst aber keine funktionelle NLS enthält.

Mit Hilfe von teilhomologen Oligonukleotidprimem wurde das UvsX-Gen aus Plasmid pExH-UvsXH6N2-2 (vergl. Abb. 1) dergestalt amplifiziert, daß am C-Terminus des entstehenden Proteins UvsXH6N2sc an Stelle der Aminosäure-Sequenz EEDTPPKKKRKVED ("nis-2", entspricht den Aminosäuren 2-15 von SEQ ID No:9 aus WO 00/40742) die Aminosäure-Sequenz SEQ ID NO:1 ("scrambled", d.h. eine durchmischte NLS-Sequenz) exprimiert wird. Letztere Aminosäuren entsprechen in ihrer Zusammenstellung, nicht aber in ihrer Reihenfolge der beschriebenen nls-2. Die Nettoladungen von UvsXH6N2-2 und UvsXH6N2sc sind somit gleich, aber nur UvsXH6N2-2 enthält ein intaktes Kemlokalisationssignal.

Das Protein UvsXH6N2sc wurde gereinigt wie für die Proteine in Beispiel 1 beschrieben.

NIH3T3-Zellen (adhärent, bis zur 70 - 80 %igen Konfluenz kultiviert) wurden mit einem Vektor, der für ein fluoreszierendes Markerprotein kodiert, transfiziert. Zu diesem Zweck wurden zunächst 25 ng Vektor-DNA in Bindungspuffer (siehe Beispiel 1) mit 1,5 mM ATP-γ-S sowie mit 16 - 18 µg der angegebenen Proteine für 30 min bei Raumtemperatur inkubiert. Die Protein-DNA-Komplexe wurden zu jeweils 3 x 10⁵ NIH3T3-Zellen, resuspendiert in 80 µl Elektroporationspuffer (140 mM Na₂HP₄/NaH₂PO₄, 10 mM MgCl₂, 5 mM KCI, pH 7,2), gegeben. Die Elektroporation erfolgte in einer Küvette mit 2 mm Elektrodenabstand über eine exponentielle Entladung bei einer Spannung von 240 V und einer Kapazität von 450 µF. Die Halbwertzeit des Spannungsabfalls betrug typischerweise 12 msec. Nach Zugabe von 400 µl Medium, zusammengesetzt aus RPMI 1640 Fa. Gibco, 5 % FCS, 2 mM Glutamax (L-Alanyl-L-Glutamin, Fa. Invitrogen), 100 U/ml Penicillin/Streptomycin, 0,5 mM β-Mercaptoethanol, wurden die Zellen in Kulturschalen (6-Loch-Platten) zu 1 ml vorgewärmtem Medium gegeben und bei 37 °C und 5 % CO2 inkubiert. Nach 6 h erfolgte die durchflußzytometrische Analyse (FACScan).

Das Ergebnis ist in Abbildung 6 grafisch dargestellt: 9 % der mit freier Vektor-DNA transfizierten Zellen exprimieren das Markerprotein. Die Expressionsrate der Zellen, die mit Vektor-UvsX-NLS (DNA+UvsXH6N2-2) transfiziert wurden, liegt dagegen bei 21 %. Im Vergleich dazu beträgt die Expressionsrate der Zellen bei einer Transfektion mit Vektor-UvsX-scrambled-NLS (DNA+UvsXH6N2sc) lediglich 4 %. UvsX, modifiziert mit einem Kerntranslokalisationssignal, führt somit zu einer deutlich erhöhten Effizienz sowohl im Vergleich mit freier DNA als auch im Vergleich mit der Modifikation durch ein nicht-funktionelles Kerntranslokalisationssignal. Da es sich bei der letztgenannten Transfektion um die eigentliche Kontrolle handelt, ergibt sich hieraus, dass durch die Modifikation des UvsX die Transfektionseffizienz auf das 5-fache gesteigert werden konnte. Durch das erfindungsgemäße Verfahren bzw. Transfektionsagens läßt sich also die Transfektionseffizienz deutlich steigem. Darüber hinaus ist durch die Modifikation des NPF-bildenden Proteins auch in vorteilhafter Weise eine gezielte Steuerung des Transfektionsvorgangs, hier beispielsweise ein Dirigieren in den Zellkern, möglich (siehe auch Beispiel 6).

### Beispiel 6: Transfektion einer Zelllinie (NIH3T3) in Kombination mit Mikroinjektion

140 ng eines 1,7 kb Expressionsvektor-DNA-Fragments wurden mit 9 µg modifiziertem UvsX-Protein wie oben beschrieben in Bindungspuffer und 1 mM ATP-γ-S in einem Endvolumen von 20 µl für 30 min bei RT inkubiert. Als Injektionsmarker diente BSA-Cy5, das unmittelbar vor der Injektion in einer Konzentration von ca. 1 µg/µl eingesetzt wurde.
Am Vortag auf CELLocate Coverslips (Eppendorf) subkonfluent ausgesäte NIH3T3-Zellen wurden mit Hilfe eines Micromanipulators und Transjektors (Eppendorf) unter einem inversen Fluoreszenzmikroskop (Leica DMIL) durch auf Femtotipps (Eppendorf) geladene Proben mikroinjiziert.
Die Auswertung erfolgte fluoreszenzmikroskopisch (Olympus BX 60-Fluoreszenzmikroskop, digitale S/W-Kamera SPOT-RT von Diagnostic Instruments Inc., Auswertungssoftware: Metaview Imaging System von Universal Imaging Corporation) nach 5 h weiterer Inkubation der Zellen bei 37 °C und 5 % CO2. Abbildung 7 zeigt mikroinjizierte NIH3T3-Zellen. Die Bilder 1 und 2 zeigen Zellen, die mit DNA und UvsXH6N2sc ins Zytoplasma injiziert wurden, die Bilder 3 und 4 solche, die mit DNA und UvsXH6N2-2, die Bilder 5 und 6 solche, die nur mit DNA injiziert wurden. Expression war nur zu beobachten, wenn die Protein-DNA-Komplexe UvsXH6N2-2, also mit einem Kerntranslokalisationssignal modifiziertes UvsX-Protein enthielten (Abb. 7, Bild 3). Selbst bei sehr langer Belichtung konnte in den Kontrollansätzen (Abb. 7, Bild 5: nur DNA bzw. Abb. 7, Bild 1: DNA mit UvsX-scrambled-NLS) bei Zellen, die bei der Mikroinjektion eindeutig nur im Zytoplasma, nicht im Kern, getroffen wurden, keine Expression beobachtet werden.

### Beispiel 7: Herstellung von rekombinanten SASP als NPF-bildende Proteine

### Klonierung, Expression und Reinigung des SASP-Proteins aus B. subtilis

Das SspC-Gen aus *Bacillus subtilis,* welches für ein SASP ("small acid-soluble spore protein") kodiert, wurde aus 8 Oligonukleotiden nach der Khorana-Methode (beschrieben in Bertram und Gassen: Gentechnische Methoden, Gustav Fischer Verlag, 1991,S. 212-213) synthetisiert und zwischen die Ncol-und Bglll-Schnittstellen des Plasmids pARA13 (Cagnon et al., 1991; Protein Engng. 4: 843-847) ligiert. Dabei wurde die Proteinsequenz mit der NCBI-Protein Accession Nr.: NP_389876 zugrunde gelegt. Die reverse Translation in DNA erfolgte unter Verwendung der in (Andersson und Kurland 1990; Microbiol. Rev. 54: 98-210) beschriebenen Codon-Präferenzen von in *E. coli* stark exprimierten Genen. Das enstehende Plasmid pARA13-SASP diente als Matrize für die Klonierung von zwei weiteren Plasmiden, welche für SASP-Proteine kodieren, die entweder N-terminal (H6-SASP) oder C-terminal (SASP-H6) eine Polyhistidinsequenz von 6 Histidinen tragen (Abb. 8). Die Plasmide wurden in den *E.coli*-Stamm BL21 (DE3) pLysS (Novagen, Madison) transformiert und auf LB/Ampicillin/Glucose (0,2%) ausplattiert.

20 ml M9-Minimalmedium (0,2% Glucose) wurden jeweils mit Einzelkolonien beimpft und über Nacht bei 37° C und 220 Upm wachsen gelassen. Am nächsten Tag wurden die Kulturen bei einer OD₆₀₀ von ca. 1,0 mit 0,2% Arabinose induziert und für weitere 6 Stunden wachsen gelassen. Rohextrakte (0,5 ml pelletierte Kultur in PBS/Ladepuffer) wurden auf hochauflösende SDS-Gele (nach Schägger und von Jagow 1987; Anal. Biochem. 166:368-79) aufgetragen und mit Coomassie-Blau gefärbt (Abb. 9 A).

Zur präparativen Aufreinigung wurden 2 I M9/Glucose 1: 200 mit einer Übernachtkultur angeimpft. Bei einer OD₆₀₀ von ca. 1,0 wurde wiederum mit 0,2% Arabinose induziert und für 6 h wachsen gelassen. Die Bakterien wurden dann pelletiert und bei -20°C eingefroren. Exemplarisch ist im Folgenden die Aufreinigung von SASP-H6 beschrieben. Nach Auftauen des Pellets (ca. 7 g) wurden diese in 14 ml Startpuffer (s. Beispiel 1), versehen mit einer Tablette Complete EDTA-free Protease Inhibitor Cocktail, Roche, Mannheim, resuspendiert und für 3 min bei 280 Watt (Labsonic U (Braun Biotech, Melsungen repeating duty puls; 0,5 sec) auf Eis sonifiziert. Der Extrakt wurde bei 4 °C zentrifugiert. Die Aufreinigung erfolgte ansonsten wie für die UvsX-Proteine beschrieben über HiTrap Chelating Columns (Amersham Pharmacia, Uppsala). Die Fraktionen zwischen 200 mM und 500 mM Imidazol, die das Protein in hoher Konzentration enthielten, wurden vereinigt und mit Hilfe von Centriplus-Säulen (YM-3, Millipore, Eschborn) auf ca. 3 ml eingeengt. Das SASP-Protein wurde dann drei Mal gegen 1 x ZI-Puffer (siehe Beispiel 1) dialysiert und Aliquots in einer Konzentration von ca. 5 µg/µl bei -80°C eingefroren.

### Test der DNA-Bindungsfähigkeit des SASP-Proteins

Jeweils 125 ng eines 1,7 kb-DNA-Fragments wurden mit verschiedenen Mengen SASP-H6-Protein in 1 x ZI-Puffer bzw. 1/10 x ZI-Puffer für 30 min. bei Raumtemperatur vorinkubiert und anschließend auf ein 0,8 %iges TAE-Agarose-Gel aufgetragen.

Abbildung 9 B zeigt, dass die DNA vollständig vom Protein gebunden wird. Das diffuse Erscheinungsbild der DNA-Protein-Banden könnte darin begründet sein, dass die Proteine möglicherweise während des Gellaufs von der DNA dissoziieren.

### Beispiel 8: Herstellung eines Transfektionsagens, basierend auf SASP als NPF-bildendem Protein mit einem Kernlokalisationssignal als Modifizierung

Zur Herstellung eines SASP mit einem Kernlokalisationssignal (NLS) als funktioneller Komponente wurde an den bereits vorhandenen Klon pARA13-SASP-H6 (siehe Abb. 8) C-terminal eine für ein Kernlokalisationssignal ("nls-2", Aminosäuren 2-15, SEQ ID NO: 9 aus WO 00/40742) kodierende DNA-Sequenz über PCR-Amplifikation angehängt. Dabei diente das oben genannte Plasmid als PCR-Matrize. Das entstehende Plasmid pARA13-SASP-H6N2 (siehe Abb. 8) wurde wie in Beispiel 7 beschrieben, transformiert und das Protein SASP-H6N2 entsprechend aufgereinigt.

### Test der DNA-Bindungsfähigkeit des SASP-NLS-Proteins

Jeweils 125 ng eines 1,7 kb-DNA-Fragments wurden mit verschiedenen Mengen SASP-H6N2-Protein in 1 x ZI-Puffer für 30 min. bei Raumtemperatur vorinkubiert und anschließend auf ein 0,8 %iges TAE-Agarose-Gel aufgetragen.
Abbildung 10 B zeigt, dass auch das NLS-modifizierte SASP die DNA retardiert. Die DNA-Protein-Banden erscheinen diffus.

### Beispiel 9: Herstellung eines Transfektionsagens mit einem Integrinbindungsmotiv für die Assoziation des Komplexes an die Zelloberfläche als funktioneller Komponente

Integrine sind membranverankerte Adhäsionsproteine auf der Zelloberfläche, von denen einige ein Peptidmotiv aus drei Aminosäuren (Arginin-Glycin-Asparaginsäure oder "RGD"-Motiv) als Bindungspartner erkennen. Die Bindung führt zur Clusterung mehrerer Integrinmoleküle auf der Zelloberfläche und zur Endozytose (Plow et al., 2001). Durch die Modifikation von UvsX als NPF-bildendem Protein mit einem RGD-Motiv kann erreicht werden, daß das Transfektionsagens spezifisch über Integrine in die endosomalen Kompartimente der Zelle aufgenommen wird.

### Aufbau der Proteine

Die verwendeten Proteine H6UvsX und UvsXH6N2-2 sind mit den in Abbildung 1 dargestellten und in den Beispielen 1 und 2 beschriebenen identisch.

Das Protein H6UvsX*(RGD2) wurde durch chemische Kopplung hergestellt. Das RGD2 genannte Peptid ist ein Nonapeptid (NITRGDTYI) aus dem Penton Base Protein des Adenovirus Typ7 (Bal et al., 2000), das so synthetisiert wurde, dass an der N-terminalen Aminogruppe eine chemisch aktive Gruppe (SMCC, Succinimidyl 4[N-Maleimidomethyl]-Cyclohexan-1-Carboxylat) steht, die die Kopplung an freie Cystein-SH-Gruppen im UvsX-Protein ermöglicht.
Für die Kopplung wurden 6 nmol H6UvsX mit 60 nmol Peptid in 76 mM K₂HPO₄, 17mM KH₂PO₄, 14mM NaH₂PO₄, pH=7,2, eine Stunde lang bei 37 °C inkubiert, anschließend durch mehrmaliges Waschen mit Inkubationspuffer über einen MicroCon-Filter (10 kDa Ausschlußgröße) von überschüssigem Peptid gereinigt. Die erfolgreiche Kopplung wurde durch ein verändertes Laufverhalten in einer SDS-Polyacrylamidgelelektrophorese nachgewiesen. Abbildung 11 zeigt zwei unabhängig voneinander hergestellte Präparationen von H6UvsX*(RGD2) auf einem SDS-Polyacrylamidgel. Das gekoppelte Peptid führt zu einer Erhöhung des Molekulargewichts und damit zur Änderung des Laufverhaltens in SDS-Gelen.

### Versuchsbeschreibung:

### Bindung von UvsX-NLS und UvsX-RGD an doppelsträngige DNA und Bildung gemischter NPFs:

Ansätze von jeweils 140 ng eines gereinigten 1.6 kb-PCR-DNA-Fragments mit einem Gemisch aus 15 µg H6UvsX*(RGD2) und 4 µg UvsXH6N2-2, mit 4 µg UvsXH6N2-2 allein und mit 15 µg H6UvsX*(RGD2) allein wurden in 76 mM K₂HPO₄, 17 mM KH₂PO₄, 14 mM NaH₂PO₄, pH=7,2, 5 mM MgCl₂ und 1 mM ATP-γ-S in einem Endvolumen von 20 µl für 30 min bei Raumtemperatur inkubiert und anschließend auf ein 0,8 %iges TAE/Agarose-Gel aufgetragen, das nachträglich mit Ethidiumbromid gefärbt wurde. Die Elektrophorese erfolgte für 1 h bei 100 V.

Abbildung 12 zeigt, dass die beiden unterschiedlich modifizierten Proteine mit der DNA jeweils NPFs bilden, die sich in ihrem Laufverhalten deutlich unterscheiden. NPFs, bestehend aus einem doppelsträngigen DNA-Fragment und einem Gemisch aus H6UvsX*(RGD2) und UvsXH6N2-2 (Spur 1), oder aus UvsXH6N2-2 allein (Spur 2) bzw. aus H6UvsX*(RGD2) allein (Spur 3), wurden in einem Agarose-Gel elektrophoretisch aufgetrennt. Da die Proteinmenge im Unterschuß vorhanden ist, liegt auch freies DNA-Fragment vor. Beide Proteine in einem Ansatz binden gemeinsam an das DNA-Fragment und führen zu einem gemischten NPF, das eine Molekularmasse zwischen den NPFs der reinen Proteine besitzt (Spur 1). Hieraus folgt also, dass UvsX-NLS und UvsX-RGD an doppelsträngige DNA binden und gemischte NPFs bilden können.

### Beispiel 10: Spezifische Aufnahme von NPFs in Zellen durch Integrin-vermittelte Endozyfose

### Aufbau der Proteine

In Analogie zu Beispiel 1 wurde Plasmid UvsXH6N2NIT-2 (Abb. 1), welches die Expression eines Fusionsproteins aus UvsX und einem Integrin-bindenden RGD-Motiv erlaubt, rekombinant hergestellt.
Aminosäuren 1-391: UvsX aus dem Phagen T4 (NCBI-Protein Accession-No.: AAD42669, Aminosäuren 1-391), Aminosäuren 392-394: Linker aus den Aminosäuren S³⁹²YG³⁹⁴, Aminosäuren 395-400: H³⁹⁵HHHHH⁴⁰⁰, Aminosäuren 401-403: Linker aus den Aminosäuren M⁴⁰¹YS⁴⁰³, Aminosäuren 404-417: Kernlokalisationssignal nls-2 (Aminosäuren 2-15, SEQ ID NO: 9 aus WO 00/40742), Aminosäuren 418-420: C-Terminus aus den Aminosäuren G⁴¹⁸YP⁴²⁰ und Aminosäuren 421-432: RGD-Motiv "NIT": N⁴²¹IT*RGD*TYIPYP⁴³².

### Versuchsbeschreibung:

### Bildung von NPFs aus Fluoreszein-markierter DNA und UvsX-Derivaten:

Jeweils 1 µg eines gereinigten 1.6 kb PCR-DNA-Fragments, das AlexaFluor488-markiertes dUTP (Molecular Probes, Eugene, Oregon, USA) anstelle von dTTP enthält, wurden in 76 mM K₂HPO₄, 17 mM KH₂PO₄, 14 mM NaH₂PO₄, pH=7,2, 5 mM MgCl₂ und 1 mM ATP-γ-S mit 100 µg gereinigtem UvsXH6N2 bzw. UvsXH6N2NIT-2 in einem Endvolumen von 200 µl für 30 min bei Raumtemperatur inkubiert. Anschließend wurden 10 µl von jedem NPF-Ansatz auf ein 0,8 %iges TAE/Agarose-Gel aufgetragen, das mit Ethidiumbromid nachträglich gefärbt wurde. Die Elektrophorese erfolgte für 1 Stunde bei 100 V. Abbildung 13 zeigt, dass die DNA vollständig retardiert wird. Die DNA-Bindung der Proteine wird durch die Fluoreszeinmarkierung der DNA also nicht behindert.

### Aufnahme der NPFs in NIH3T3-Zellen durch Endozytose:

NIH3T3 Zellen wurden in 6-Loch-Platten (3×10⁵ pro Loch) ausgesät, über Nacht bei 37 °C und 5 % CO₂ inkubiert und am nächsten Morgen mit vorgewärmtem FCS-freiem Medium gewaschen, dann mit 2 ml FCS-freiem Medium versetzt. Dazu wurden 190 µl der NPF-Ansätze (s.o.) gegeben, 30 min bei Raumtemperatur inkubiert, der Überstand abgenommen, die Zellen gewaschen und mit 3 ml Medium (mit 10% FCS) überschichtet. Nach einer weiteren 1-stündigen Inkubation bei 37 °C im Brutschrank erfolgte die Auswertung unter dem Fluoreszenzmikroskop. In den Abbildungen 14 a und 14 b sind jeweils eine Aufnahme im Hellfeld (unten) und eine in Auflichtfluoreszenz (oben) gezeigt.

Zu erkennen sind im Hellfeld vesikuläre intrazelluläre Kompartimente, die im Fluoreszenzlicht aufgrund der endozytierten DNA aufleuchten (Abb. 14 a, b oben).

Es wurden mehrere Aufnahmen von jedem Loch gemacht. Die Zellen wurden gezählt und der prozentuale Anteil von Zellen bestimmt, die mindestens ein fluoreszierendes vesikuläres Kompartiment enthielten (dargestellt in Abbildung 15). Jede Aufnahme zeigte durchschnittlich 35 Zellen. Neun Aufnahmen wurden von den Ansätzen mit UvsXH6N2NIT-2 und fünf Aufnahmen von den Ansätzen mit UvsXH6N2-2 ausgewertet.

Das Ergebnis zeigt, dass die Modifikation von UvsX mit einem Integrinbindungsmotiv als funktioneller Komponente (UvsXH6N2-NIT-2) im Vergleich zur Kontrolle (UvsXH6N2-2) eine deutlich erhöhte endozytotische Aufnahme des Transfektionsagens in die Zellen bewirkt.

### Beispiel 11: Herstellung eines Transfektionsagens, basierend auf hRad51 als NPF-bildendem Protein

Als NPF-bildende Proteine wurden die Proteine hRad51H6, und hRad51H6N2 verwendet (siehe Abbildung 16).

### Aufbau der Proteine:

### hRad51H6 (352 Aminosäuren):

Aminosäuren 1-339: humanes Rad51 (NCBI-Protein Accession-No.: Q06609, Aminosäuren 1-339), Aminosäuren 340-343: Linker aus den Aminosäuren Y³⁴⁰SYG³⁴³, Aminosäuren 344-349: H³⁴⁴HHHHH³⁴⁹ für die Aufreinigung über Nickel-Chelat-Affinitätschromatografie, Aminosäuren 350-352: C-Terminus aus den Aminosäuren M³⁵⁰YS³⁵².

### hRad51H6N2 (369 Aminosäuren):

Aminosäuren 1-339: humanes Rad51 (NCBI-Protein Accession-No.: Q06609, Aminosäuren 1-339), Aminosäuren 340-343: Linker aus den Aminosäuren Y³⁴⁰SYG³⁴³, Aminosäuren 344-349: H³⁴⁴HHHHH³⁴⁹ für die Aufreinigung über Nickel-Chelat-Affinitätschromatografie, Aminosäuren 350-352: Linker aus den Aminosäuren M³⁵⁰YS³⁵²,, Aminosäuren 353-366: Kernlokalisationssignal nls-2 (Aminosäuren 2-15, SEQ ID NO: 9 aus WO 00/40742), Aminosäuren 367-369: C-Terminus aus den Aminosäuren G³⁶⁷YP³⁶⁹.

### Klonierung der Expressionsplasmide

Für die Expression der oben genannten Proteine in geeigneten *Escherichia coli* Zellen wurden Plasmide konstruiert, die eine kodierende Sequenz für hRad51H6, bzw. hRad51H6N2 unter der Kontrolle des lac-Promoters enthalten (pExH-hRad51H6 bzw. pExH-hRad51H6N2, siehe Abb. 16). Als Ausgangsplasmide wurden pExH-UvsXH6-2 und pExH-UvsXH6N2-2 verwendet (siehe Abb. 16). Die kodierende Region für UvsX wurde jeweils mit EcoR V und BsiW I herausgeschnitten und durch ein ebenso geschnittenes PCR-Fragment mit der kodierenden Region für hRad51 ersetzt. Diese wurde aus einer humanen cDNA-Bibliothek mt hRad51-spezifischen Primem amplifiziert, die die gewünschten Restriktionsschnittstellen enthalten. Die PCR-Produkte enthielten am 5'-Ende vor dem Startcodon die zusätzlichen Nukleotide 5'-CACACATCTAGACGTACGGATATCAT-3', sowie am 3'-Ende anstelle des Stopcodons die zusätzlichen Nukleotide 5'-TACTCGTACGGAGGTGGCGGCCGCTGTGTG-3'.

### Reinigung:

Eine Vorkultur von 5 ml dYT/Ampicillin (100µg/ml) wurde mit einer Kolonie von pExH-Rad51H6 bzw. pExH-Rad51H6N2 in DH5 angeimpft und 5 Stunden bei 37° C mit 250 Upm wachsen gelassen. 10 I dYT/Ampicillin (100µg/ml) wurden mit dieser Vorkultur angeimpft und für weitere 24 Stunden bei 37° C mit 210 UPM wachsen gelassen. Die bei 7000 x g geernteten Kulturen ergaben ca. 30-50 g Bakteriensediment. Dieses wurde für 1-3 Tage bei -20° C eingefroren. Das Sediment wurde auf Eis getaut und in 100 ml kaltem Start-Puffer resuspendiert. Die Zellen wurden daraufhin mit Ultraschall unter Verwendung eines B. Braun Labsonic U aufgeschlossen (große Sonde, Parameter: 300W, 0,5 sec Pulsdauer pro Sekunde, 8 Minuten Beschallung). Anschließend wurde bei 4° C mit 10 mg Lysozym (Serva, 190.000 u/mg) für 1 h und nach Zugabe von 50 µl DNAse I (Serva, 2 mg/ml) weitere 30 min unter langsamen Rühren inkubiert. Nach Abzentrifugieren des Lysats (45 min 18000 x g, 4 °C) wurde der Überstand durch Sterilfilter (Porengröße 0,45 µm und 0,2 µm) filtriert und auf eine mit Ni⁺⁺-Ionen beladene und äquilibrierte 1 ml HiTrapTM Chelating-Säule (Pharmacia) geladen. Die weiteren Aufreinigungsschritte erfolgten nach dem entsprechenden Pharmacia-Protokoll für Proteine, die mit einem Histidin-Hexamer versehen wurden. Aliquots der verschiedenen Elutionsfraktionen wurden auf SDS/C-oomassie-Gelen aufgetragen. Die jeweils saubersten Fraktionen wurden vereinigt und über Centriplus YM30- Säulen (Millipore) nach dem entsprechenden Protokoll weiter eingeengt. Danach erfolgte eine zweimalige Dialyse (Dialyseschlauch: Spectra/Por, MWCO: 25.000) gegen ein mindestens eintausendfaches Volumen ZI-Puffer für jeweils mindestens eine Stunde bei 4° C, dann über Nacht bei 4° C gegen ZI-Puffer/50 % Glyzerin. Das Dialysat wurde in 30-50 µI-Fraktionen aliquotiert und bei -80° C aufbewahrt. Abb. 17 A zeigt die gereinigten hRad51-H6 und hRad51-H6N2 Proteine.

### Verwendete Puffer:

Entsprechend Beispiel 1, Elutionspuffer abweichend: 20 mM Pi, 0,5 M NaCl, 100-1000 mM Imidazol, pH=7,4

### Konzentrationsbestimmung:

Die Konzentration der hRad51-Proteine wurde durch Messen der OD₂₈₀ unter Einbeziehung des über das Programm Gene InspectorTM (Textco, Inc.) errechneten Extinktionskoeffizienten bestimmt.
Bei hRad51H6 und hRad51H6N2 betrug sie 11-13 µg/µl.

### Versuchsbeschreibung:

### Bindung von NLS-modifiziertem hRad51 an doppelsträngige DNA:

Über Ni⁺⁺-Sepharose gereinigtes hRad51H6N2 wurde mit jeweils 100 ng eines 0,9 kb PCR-Fragmentes inkubiert.
Ein durch Proteinbindung verursachter Shift der DNA im Agarosegel zeigt, dass hRad51H6N2 in Abhängigkeit von der Konzentration, kooperativ doppelsträngige DNA bindet (Abb. 17B). Schon bei geringen Mengen an hRad51H6N2 werden einzelne DNA-Moleküle vollständig von hRad51H6N2 gebunden und daher maximal retardiert, so dass bei Erhöhung der Proteinmenge die Retardierung der DNA nicht noch weiter zunimmt. Hieraus folgt, dass hRad51H6N2 an dsDNA bindet.

### Beispiel 12: Herstellung eines Transfektionsagens, basierend auf UvsX, mit einem Signal für den nicht-endosomalen Membrandurchtritt und einem Kernlokalisationssignal als funktionelle Komponenten

In Analogie zu Beispiel 1 wurde ein Plasmid hergestellt, das die Expression des Fusionsproteins UvsXH6N2VP22c50 (siehe Abb. 1) erlaubt, das ausgehend von den im Beispiel 2 beschriebenen Protein UvsXH6N2-2 zusätzlich ein Teilstück des Tegumentproteins VP22 (Gen UL49) aus dem humanen Herpesvirus 1 enthält. Das hier verwendete VP22-Peptid wirkt als Signal für den nicht-enodosomalen Durchtritt durch die Zellmembran. Das Fusionsprotein UvsXH6N2VP22c50 enthält also zusätzlich zu einem Kernlokalisationssignal (NLS) noch ein Membrantransduktionssignal.

### Aufbau des Proteins:

### UvsXH6N2VP22c50 (474 Aminosäuren):

Aminosäuren 1-391: UvsX aus dem Phagen T4 (NCBI-Protein Accession-No.: AAD42669, Aminosäuren 1-391), Aminosäuren 392-394: Linker aus den Aminosäuren S³⁹²YG³⁹⁴, Aminosäuren 395-400: H³⁹⁵HHHHH⁴⁰⁰, Aminosäuren 401-403: Linker aus den Aminosäuren M⁴⁰¹YS⁴⁰³, Aminosäuren 404-417: Kernlokalisationssignal nls-2 (Aminosäuren 2-15, SEQ ID NO: 9 aus WO 00/40742), Aminosäuren 418-422: Linker aus den Aminosäuren G⁴¹⁸YPGS⁴²², Aminosäuren 423-472: Teilstück des Tegumentproteins VP22 (Gen UL49) des humanen Herpesvirus 1 (NCBI_Protein Accession-No.: NP_044651, Aminosäuren 252-301), Aminosäuren 473-474: C-Terminus aus den Aminosäuren P⁴⁷³R⁴⁷⁴.

### Klonierung des Expressionsplasmids

Für die Expression in geeigneten *Escherichia coli* Zellen wurde pExHUvsXH6N2-2 (siehe Beispiel 1 und Abb. 1) durch Restriktionsverdauung mit Acc65 I und Spe I geöffnet und mit einem Acc65 I und Nhe I geschnittenen PCR-Produkt ligiert, das zusätzlich zu der kodierenden Sequenz für die letzten 50 Aminosäuren aus dem Tegumentprotein VP22 (Gen UL49) des humanen Herpesvirus 1 (NCBI-Nucleotide Accession-No.: NC_001806, Komplementärsequenz der Nukleotide 105486-106391), am 5'-Ende die zusätzlichen Nukleotide 5'-CACACAGGTACCCGGGATCC-3', sowie am 3'-Ende die zusätzlichen Nukleotide 5'-CCTAGGTAATAATAAGCGGCCGCGCTAGCTGTGTG-3' enthielt (siehe Abbildung 1).

### Reinigung:

Die Reinigung von UvsXH6N2VP22c50 erfolgte wie unter Beispiel 1 für H6UvsX beschrieben (siehe Abb. 18A).

Konzentration: 1,8 µg/µl.

### Versuchsbeschreibung:

### Bindung einer Mischung aus verschieden modifizierten UvsX (UvsX-NLS-VP22 und UvsX-NLS) an doppelsträngige DNA:

Jeweils 140 ng eines gereinigten 1,7 kb PCR-Fragments wurden in 96 mM K₂HPO₄, 21,5mM KH₂PO₄, 18mM NaH₂PO₄. pH=7,2, 5mM MgCl₂ und 1,3 mM ATP-γ-S mit den Mengen gemäß Abb. 18B an gereinigtem UvsXH6N2VP22c50 bzw. UvsXH6N2-2 für 30 min bei Raumtemperatur inkubiert und anschließend alle Ansätze auf ein 0,8 %iges TAE/Agarose-Gel, das mit Ethidiumbromid nachträglich gefärbt wurde, aufgetragen. Die zwei Proteine unterscheiden sich in ihrem Molekulargewicht und ihrer Nettoladung und retardieren DNA daher unterschiedlich stark während der Elektrophorese, wobei der Komplex mit UvsXH6N2VP22c50 in der Geltasche hängenbleibt und gar nicht mehr wandert (siehe Spalten 1 und 7 von Abbildung 18B). Mischt man die Proteine vor der Zugabe zur DNA, so entstehen je nach Verhältnis von UvsXH6N2-2 zu UvsXH6N2VP22c50 intermediäre Komplexe, deren Wanderungsverhalten zwischen denen der ungemischten Komplexe liegt (Abb. 18B). Dies zeigt, daß die DNA mit beiden Proteinen beladen ist. Eine Mischung verschieden, ein-oder mehrfach modifizierter NPF-bildender Proteine ist also möglich.

### Beispiel 13: Transfektion einer Zelllinie (NIH3T3) mit Komplexen aus DNA und einer Mischung aus UvsX-NLS-VP22 und UvsX-NLS

### Versuchsbeschreibung:

2,5 x 10⁵ Zellen (NIH3T3) wurden in jede Vertiefung einer 6-Loch-Platte ausgesät und am Folgetag mit einem Vektor, der ein Gen für die Expression eines fluoreszenten Reporterproteins enthält, transfiziert. Hierzu wurde 0 µg - 1 µg lineare bzw. 1 µg zirkuläre DNA mit 36 µg UvsXH6N2VP22c50 bzw. einer Mischung aus 19 µg UvsXH6N2VP22c50 und 39 µg UvsXH6N2-2 in Bindungspuffer (76 mM K₂HPO₄, 17 mM KH₂PO₄, 14 mM NaH₂PO₄, 5 mM MgCl₂, 1 mM ATP-γ-S, pH 7,21) für 30 Minuten bei Raumtemperatur vorinkubiert und zusammen mit 1 ml RPMI auf Zellen gegeben, die vorher einmal mit PBS/BSA gewaschen wurden. Nach 1 h Inkubation bei 37°C, 5% CO₂ im Brutschrank wurde jeweils 1 ml RPMI/20% FCS hinzugegeben und weiter im Brutschrank inkubiert. Die Zellen wurden daraufhin nach 4 h bzw. 24 h im Fluoreszenzmikroskop analysiert.
Dabei wurden Zellen beobachtet, die nach der Behandlung mit Komplexen aus linearer bzw. zirkulärer DNA und der Mischung aus UvsXH6N2VP22c50 und UvsXH6N2-2 das Reportergen exprimierten (siehe Abb. 19). Die Anzahl der transfizierten Zellen stieg dabei mit der Menge an eingesetzer DNA bzw. DNA-Proteinkomplexen.
Dagegen wurde keine Expression des Reportergens erzielt mit DNA-Komplexen, die nur UvsXH6N2VP22c50 enthielten, oder in Abwesenheit von DNA.

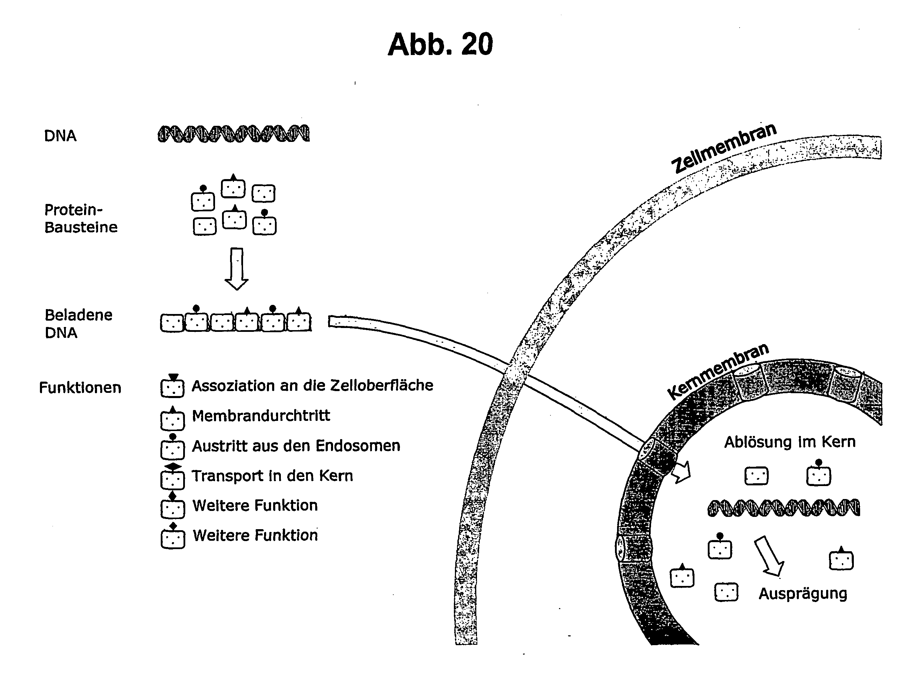

Es zeigt sich also, dass durch die Modifikation eines-NPF-bildenden Proteins, hier UvsX, mit einem membranaktiven Peptid, hier VP22, die Transfektion von Zellen und hier insbesondere der Membrandurchtritt, ermöglicht wird. Der modulare Charakter des erfindungsgemäßen Verfahrens bzw. Transfektionsagens wird durch die Kombination unterschiedlich modifizierter Proteine, hier Modifikation mit VP22 und NLS, verdeutlicht (siehe auch Abb. 20). Während das mit VP22 modifizierte UvsX den nicht-endosomalen Membrandurchtritt ermöglicht, wird die transfizierte DNA durch das mit dem NLS modifizierte UvsX vom Zytoplasma in den Zellkern dirigiert und kann anschließend dort exprimiert werden.

Es können also einzelne Schritte des komplexen Transfektionsvorgangs in besonders vorteilhafter Weise spezifisch, flexibel und mit hoher Effizienz gesteuert werden.

### Literatur

Andersson, K. (1990). Codon Preferences in Free-Living Microorganisma. Micobiol Rev 54, 98-210.
Bal, H. P., Chroboczek, J., Schoehn, G., Ruigrok, R. W., and Dewhurst, S. (2000). Adenovirus type 7 penton purification of soluble pentamers from Escherichia coli and development of an integrin-dependerit gene delivery system. Eur J Biochem 267, 6074-6081.
Baumann, P., and West, S. C. (1998). Role of the human RAD51 protein in homologous recombination and double-stranded-break repair. Trends Biochem Sci 23, 247-251.
Bianco, P. R., Tracy, R. B., and Kowalczykowski, S. C. (1998). DNA strand exchange proteins: a biochemical and physical comparison. Front Biosci 3, D570-603.
Boulikas, T. (1993). Nuclear localization signals (NLS). Crit Rev Eukaryot Gene Expr 3, 193-227.
Boulikas, T. (1996). Nuclear import of protein kinases and cyclins. J Cell Biochem 60, 61-82.
Boulikas, T. (1997). Nuclear import of DNA repair proteins. Anticancer Res 17, 843-863.
Cagnon, C., Valverde, V., and Masson, J. M. (1991). A new family of sugar-inducible expression vectors for Escherichia coli. Protein Eng 4, 843-847.
Cerutti, H., Osman, M., Grandoni, P., and Jagendorf, A. T. (1992). A homolog of Escherichia coli RecA protein in plastids of higher plants. Proc Natl Acad Sci U S A 89, 8068-8072.
Collins, L., Sawyer, G. J., Zhang, X. H., Gustafsson, K., and Fabre, J. W. (2000). In vitro investigation of factors important for the delivery of an integrin-targeted nonviral DNA vector in organ transplantation. Transplantation 69, 1168-1176.
Delcayre, A. X., Salas, F., Mathur, S., Kovats, K., Lotz, M., and Lernhardt, W. (1991). Epstein Barr virus/complement C3d receptor is an interferon alpha receptor. Embo J 10, 919-926.
Di Capua, E., Engel, A., Stasiak, A., and Koller, T. (1982). Characterization of complexes between recA protein and duplex DNA by electron microscopy. J Mol Biol 157, 87-103.
Ellouze, C., Selmane, T., Kim, H. K., Tuite, E., Norden, B., Mortensen, K., and Takahashi, M. (1999). Difference between active and inactive nucleotide cofactors in the effect on the DNA binding and the helical structure of RecA filament dissociation of RecA-DNA complex by inactive nucleotides. Eur J Biochem 262, 88-94.
Evan, G. I., Lewis, G. K., Ramsay, G., and Bishop, J. M. (1985). Isolation of monoclonal antibodies specific for human c-myc proto- oncogene product. Mol Cell Biol 5, 3610-3616.
Feero, W. G., Li, S., Rosenblatt, J. D., Sirianni, N., Morgan, J. E., Partridge, T. A., Huang, L., and Hoffman, E. P. (1997). Selection and use of ligands for receptor-mediated gene delivery to myogenic cells. Gene Ther 4, 664-674.
Feldherr, C. M., and Akin, D. (1997). The location of the transport gate in the nuclear pore complex. J Cell Sci 110 (Pt 24), 3065-3070.
Fominaya, J., and Wels, W. (1996). Target cell-specific DNA transfer mediated by a chimeric multidomain protein. Novel non-viral gene delivery system. J Biol Chem 271, 10560-10568.
Griffith, J., Makhov, A., Santiago-Lara, L., and Setlow, P. (1994). Electron microscopic studies of the interaction between a Bacillus subtilis alpha/beta-type small, acid-soluble spore protein with DNA: protein binding is cooperative, stiffens the DNA, and induces negative supercoiling. Proc Natl Acad Sci U S A 91, 8224-8228.
Harbottle, R. P., Cooper, R. G., Hart, S. L., Ladhoff, A., McKay, T., Knight, A. M., Wagner, E., Miller, A. D., and Coutelle, C. (1998). An RGD-oligolysine peptide: a prototype construct for integrin-mediated gene delivery. Hum Gene Ther 9, 1037-1047.
Hong, S. S., Gay, B., Karayan, L., Dabauvalle, M. C., and Boulanger, P. (1999). Cellular uptake and nuclear delivery of recombinant adenovirus penton base. Virology 262, 163-177.
Karlin, S., and Brocchieri, L. (1996). Evolutionary conservation of RecA genes in relation to protein structure and function. J Bacteriol 178, 1881-1894.
Karlin, S., Weinstock, G. M., and Brendel, V. (1995). Bacterial classifications derived from recA protein sequence comparisons. J Bacteriol 177, 6881-6893.
Knight, K. L., and McEntee, K. (1985). Affinity labeling of a tyrosine residue in the ATP binding site of the recA protein from Escherichia coli with 5'-p-fluorosulfonylbenzoyladenosine. J Biol Chem 260, 10177-10184.
Kukowska-Latallo, J. F., Bielinska, A. U., Johnson, J., Spindler, R., Tomalia, D. A., and Baker, J. R., Jr. (1996). Efficient transfer of genetic material into mammalian cells using Starburst polyamidoamine dendrimers. Proc Natl Acad Sci U S A 93, 4897-4902.
Lee, C. K., and Knipe, D. M. (1985). An immunoassay for the study of DNA-binding activities of herpes simplex virus protein ICP8. J Virol 54, 731-738.
Masson, J. Y., Davies, A. A., Hajibagheri, N., Van Dyck, E., Benson, F. E., Stasiak, A. Z., Stasiak, A., and West, S. C. (1999). The meiosis-specific recombinase hOmc1 forms ring structures and interacts with hRad51. Embo J 18, 6552-6560.
Mengaud, J., Ohayon, H., Gounon, P., Mege, R. M., and Cossart, P. (1996). E-cadherin is the receptor for internalin, a surface protein required for entry of L. monocytogenes into epithelial cells. Cell 84, 923-932.
Midoux, P., Mendes, C., Legrand, A., Raimond, J., Mayer, R., Monsigny, M., and Roche, A. C. (1993). Specific gene transfer mediated by lactosylated poly-L-lysine into hepatoma cells. Nucleic Acids Res 21, 871-878.
Mosig, G. (1987). The essential role of recombination in phage T4 growth. Annu Rev Genet 21, 347-371.
Neumann, G., Castrucci, M. R., and Kawaoka, Y. (1997). Nuclear import and export of influenza virus nucleoprotein. J Virol 71, 9690-9700.
Ogawa, T., Shinohara, A., Nabetani, A., Ikeya, T., Yu, X., Egelman, E. H., and Ogawa, H. (1993). RecA-like recombination proteins in eukaryotes: functions and structures of RAD51 genes. Cold Spring Harb Symp Quant Biol 58, 567-576.
Ohno, K., Sawai, K., lijima, Y., Levin, B., and Meruelo, D. (1997). Cell-specific targeting of Sindbis virus vectors displaying IgG-binding domains of protein A. Nat Biotechnol 15, 763-767.
Pack, D. W., Putnam, D., and Langer, R. (2000). Design of imidazole-containing endosomolytic biopolymers for gene delivery. Biotechnol Bioeng 67, 217-223.
Plow, E. F., Haas, T. A., Zhang, L., Loftus, J., and Smith, J. W. (2000). Ligand binding to integrins. J Biol Chem 275, 21785-21788.
Pooga, M., Soomets, U., Hallbrink, M., Valkna, A., Saar, K., Rezaei, K., Kahl, U., Hao, J. X., Xu, X. J., Wiesenfeld-Hallin, Z., et al. (1998). Cell penetrating PNA constructs regulate galanin receptor levels and modify pain transmission in vivo. Nat Biotechnol 16, 857-861.
Provoda, C. J., and Lee, K. D. (2000). Bacterial pore-forming hemolysins and their use in the cytosolic delivery of macromolecules. Adv Drug Deliv Rev 41, 209-221.
Richardson, S., Ferruti, P., and Duncan, R. (1999). Poly(amidoamine)s as potential endosomolytic polymers: evaluation in vitro and body distribution in normal and tumour-bearing animals. J Drug Target 6, 391-404.
Roca, A. I., and Cox, M. M. (1990). The RecA protein: structure and function. Crit Rev Biochem Mol Biol 25, 415-456.
Rosenkranz, A. A., Yachmenev, S. V., Jans, D. A., Serebryakova, N. V., Murav'ev, V. I., Peters, R., and Sobolev, A. S. (1992). Receptor-mediated endocytosis and nuclear transport of a transfecting DNA construct. Exp Cell Res 199, 323-329.
Sandler, S. J., Satin, L. H., Samra, H. S., and Clark, A. J. (1996). recA-like genes from three archaean species with putative protein products similar to Rad51 and Dmc1 proteins of the yeast Saccharomyces cerevisiae. Nucleic Acids Res 24, 2125-2132.
Schagger, H., and von Jagow, G. (1987). Tricine-Sodium Dodecyl Sulfate-Polyacrylamide Gel Electrophoresis for the Separation of Proteins in the Range from 1 to 100 kDa. Anal Biochem 166, 368-379.
Schoeman, R., Joubert, D., Ariatti, M., and Hawtrey, A. O. (1995). Further studies on targeted DNA transfer to cells using a highly efficient delivery system of biotinylated transferrin and biotinylated polylysine complexed to streptavidin. J Drug Target 2, 509-516.
Seitz, E. M., Brockman, J. P., Sandler, S. J., Clark, A. J., and Kowalczykowski, S. C. (1998). RadA protein is an archaeal RecA protein homolog that catalyzes DNA strand exchange. Genes Dev 12, 1248-1253.
Steinhauer, D. A., Wharton, S. A., Skehel, J. J., and Wiley, D. C. (1995). Studies of the membrane fusion activities of fusion peptide mutants of influenza virus hemagglutinin. J Virol 69, 6643-6651.
Surdej, P., and Jacobs-Lorena, M. (1994). Strategy for epitope tagging the proteincoding region of any gene. Biotechniques 17, 560-565.
Tang, M. X., and Szoka, F. C. (1997). The influence of polymer structure on the interactions of cationic polymers with DNA and morphology of the resulting complexes. Gene Ther 4, 823-832.
Thoren, P. E., Persson, D., Karlsson, M., and Norden, B. (2000). The antennapedia peptide penetratin translocates across lipid bilayers - the first direct observation. FEBS Lett 482, 265-268.
Thyagarajan, B., Padua, R. A., and Campbell, C. (1996). Mammalian mitochondria possess homologous DNA recombination activity. J Biol Chem 271, 27536-27543.
Wagner, E. (1999). Application of membrane-active peptides for nonviral gene delivery. Adv Drug Deliv Rev 38, 279-289.
Wang, P., Palese, P., and O'Neill, R. E. (1997). The NPI-1/NPI-3 (karyopherin alpha) binding site on the influenza a virus nucleoprotein NP is a nonconventional nuclear localization signal. J Virol 71, 1850-1856.
Weisbart, R. H., Baldwin, R., Huh, B., Zack, D. J., and Nishimura, R. (2000). Novel protein transfection of primary rat cortical neurons using an antibody that penetrates living cells. J Immunol 164, 6020-6026.
Yamada, M., and Kasamatsu, H. (1993). Role of nuclear pore complex in simian virus 40 nuclear targeting. J Virol 67, 119-130.
Yu, X., and Egelman, E. H. (1993). DNA conformation induced by the bacteriophage T4 UvsX protein appears identical to the conformation induced by the Escherichia coli RecA protein. J Mol Biol 232, 1-4.
Zauner, W., Blaas, D., Kuechler, E., and Wagner, E. (1995). Rhinovirus-mediated endosomal release of transfection complexes. J Virol 69, 1085-1092.

## Patentansprüche

1. Transfektionsagens enthaltend ein Nukleoproteinfilament, gebildet aus mindestens einer zu transfizierenden Nukleinsäure und mindestens einem zur Nukleoproteinfilamentbildung fähigen Protein, **dadurch gekennzeichnet, dass** das zur Nukleoproteinfilamentbildung fähige Protein mit mindestens zwei funktionellen Komponenten unterschiedlicher Funktion modifiziert ist, wobei eine funktionelle Komponente ein Kernlokalisationssignal ist und mindestens eine weitere funktionelle Komponente ausgewählt ist aus der Gruppe bestehend aus einem VP22-Peptid und einem Integrin-bindenden RGD-Motiv.

2. Transfektionsagens enthaltend ein Nukleoproteinfilament, gebildet aus mindestens einer zu transfizierenden Nukleinsäure und zur Nukleoproteinfilamentbildung fähigen Proteinen, **dadurch gekennzeichnet, dass** mindestens zwei gleiche oder unterschiedliche, zur Nukleoproteinfilamentbildung fähige Proteine mit einer Komponente unterschiedlicher Funktion modifiziert sind, wobei eine funktionelle Komponente ein Kernlokalisationssignal ist und mindestens eine weitere funktionelle Komponente ausgewählt ist aus der Gruppe bestehend aus einem VP22-Peptid und einem Integrin-bindenden RGD-Motiv.

3. Transfektionsagens nach Anspruch 1 oder 2, **dadurch gekennzeichnet, dass** es als filamentbildendes Protein ein Protein, ausgewählt aus der Gruppe der Proteine RecA, RadA, UvsX, RAD51, hDmc1, SASP, ICP8, vorzugsweise UvsX, besonders bevorzugt hRAD51, oder eine Mischung aus mindestens 2 der aufgezählten Proteinen enthält.

4. Verwendung eines Transfektionsagens nach einem der Ansprüche 1 bis 3 zur Herstellung eines Medikaments zur gentherapeutischen Behandlung von Menschen und Tieren.

5. Pharmazeutische Zubereitung, **gekennzeichnet durch** den Gehalt an einem Transfektionsagens nach einem der Ansprüche 1 bis 3.

6. Kit, geeignet zur Transfektion von Zellen mit Nukleinsäuren, umfassend modifizierte zur Nukleoproteinfilamentbildung fähige Proteine wie definiert in einem der Ansprüche 1 bis 3 sowie mindestens eine der folgenden Komponenten:
a) Nukleosidtriphosphat und/oder Nukleosidtriphosphat-Analoga
b) mindestens eine zu transfizierende Nukleinsäure
c) Hilfs- und Zusatzstoffe

7. Verfahren zur *in vitro* Transfektion von Zellen mit Hilfe zumindest eines Transfektionsagens nach einem oder mehreren der Ansprüche 1 bis 3, bei dem das Transfektionsagens auf die zu transfizierenden Zellen gegeben wird.

8. Verfahren nach Anspruch 7, **dadurch gekennzeichnet, dass** der Komplex durch Zugabe von Nukleosidtriphosphaten und/oder nicht-hydrolysierbaren Analoga davon, insbesondere durch ATP (Adenosintriphosphat) und/oder GTP (Guanosintriphosphat) und/oder deren nicht-hydrolysierbarer Analoga, stabilisiert wird.

9. Verfahren nach Anspruch 7 oder 8, **dadurch gekennzeichnet, dass** dieses in Kombination mit anderen biologischen und/oder chemischen und/oder physikalischen Transfektionsmethoden für Nukleinsäuren, insbesondere in Kombination mit Elektroporation, angewendet wird.

## Claims

1. Transfection agent containing a nucleoprotein filament and being formed of at least one nucleic acid to be transfected and at least one protein being capable of forming nucleoprotein filaments, **characterised in that** the protein that is capable of forming nucleoprotein filaments is modified with at least two functional components having different functions, wherein one functional component is a nuclear localisation signal and at least one further functional component is selected from the group consisting of a VP22 peptide and an Integrin-binding RGD motif.

2. Transfection agent containing a nucleoprotein filament and being formed of at least one nucleic acid to be transfected and proteins being capable of forming nucleoprotein filaments, **characterised in that** at least two identical or different proteins that are capable of forming nucleoprotein filaments are modified with a functional component having different functions, wherein one functional component is a nuclear localisation signal and at least one further functional component is selected from the group consisting of a VP22 peptide and an Integrin-binding RGD motif.

3. Transfection agent according to claim 1 or 2, **characterised in that** it contains, as the filament-forming protein, a protein selected from the group of the proteins RecA, RadA, UvsX, RAD51, hOmc1, SASP, ICP8, preferably UvsX, more preferably hRAD51, or a mixture of at least 2 of the listed proteins.

4. Use of a transfection agent according to any one of the claims 1 to 3 for producing a medicament for gene therapeutic treatment of humans and animals.

5. Pharmaceutical preparation **characterised by** containing a transfection agent according to any one of the claims 1 to 3.

6. Kit, suitable for transfection of cells with nucleic acids, comprising modified proteins capable of forming nucleoprotein filaments as defined in any one of the claims 1 to 3 and at least one of the following components:
a) nucleoside triphosphate and/or nucleoside triphosphate analogues
b) at least one nucleic acid to be transfected
c) adjuvants and additives

7. Method for transfecting cells *in vitro* using at least one transfection agent according to any one of the claims 1 to 3, wherein the transfection agent is added to the cells to be transfected.

8. Method according to claim 7, **characterised in that** the complex is stabilized by addition of nucleoside triphosphates and/or non-hydrolyzable analogues thereof, especially by ATP (adenosintriphosphate) and/or GTP (guanosintriphosphate) and/or their non-hydrolyzable analogues.

9. Method according to claim 7 or 8, **characterised in that** the method is employed in combination with other biological and/or chemical and/or physical transfection methods for nucleic acids, especially in combination with electroporation.

## Revendications

1. Agent de transfection contenant un filament de nucléoprotéine, formé d'au moins un acide nucléique à transfecter et d'au moins une protéine apte à la formation du filament de nucléoprotéine, **caractérisé en ce que** la protéine apte à la formation du filament de nucléoprotéine est modifiée à l'aide d'au moins deux composants fonctionnels à fonction différente, un composant fonctionnel étant un signal de localisation de noyau et au moins un autre composant fonctionnel étant sélectionné dans le groupe composé d'un peptide VP22 et d'un motif RGD formant de l'intégrine.

2. Agent de transfection contenant un filament de nucléoprotéine, formé d'au moins un acide nucléique à transfecter et de protéines aptes à la formation du filament de nucléoprotéine, **caractérisé en ce qu'**au moins deux protéines identiques ou différentes aptes à la formation du filament de nucléoprotéine sont modifiées à l'aide d'un composant à fonction différente, un composant fonctionnel étant un signal de localisation de noyau et au moins un autre composant fonctionnel étant sélectionné dans le groupe composé d'un peptide VP22 et d'un motif RGD formant de l'intégrine.

3. Agent de transfection selon la revendication 1 ou 2, **caractérisé en ce qu'**il contient comme protéine formant du filament de nucléoprotéine une protéine sélectionnée dans le groupe composé des protéines RecA, RadA, UsvX, RAD51, hDmc1, SASP, ICP8, de préférence UvX, de manière particulièrement préférentielle hRAD51, ou un mélange d'au moins 2 des protéines énumérées.

4. Utilisation d'un agent de transfection selon une des revendications 1 à 3 pour la production d'un médicament de traitement par thérapie génique destiné à l'homme et à l'animal.

5. Préparation pharmaceutique, **caractérisée par** sa teneur en agent de transfection selon une des revendications 1 à 3.

6. Kit adapté pour la transfection de cellules avec des acides nucléiques, comprenant des protéines modifiées aptes à la formation de filament de nucléoprotéine telles que définies dans une des revendications 1 à 3 ainsi qu'au moins un des composants suivantes :
a) du triphosphate de nucléoside et/ou un analogue au triphosphate de nucléoside,
b) au moins un acide nucléique à transfecter,
c) des substances auxiliaires et additives.

7. Procédé pour la transfection *in vitro* de cellules à l'aide d'au moins un agent de transfection selon une ou plusieurs des revendications 1 à 3, dans lequel l'agent de transfection est mis sur les cellules à transfecter.

8. Procédé selon la revendication 7, **caractérisé en ce que** le complexe est stabilisé par ajout de triphosphates de nucléotide et/ou de leurs analogues non hydrolisables, notamment par de l'ATP (adénosinetriphosphate) et/ou du GTP (guanosinetriphosphate) et/ou leurs analogues non hydrolisables.

9. Procédé selon la revendication 7 ou 8, **caractérisé en ce que** celui-ci est appliqué en combinaison avec d'autres méthodes de transfection biologiques et/ou chimiques et/ou physiques pour les acides nucléiques, en particulier en combinaison avec une électroporation.
